(19) Europäisches Patentamt
European Patent Office
Office européen des brevets

(11) Veröffentlichungsnummer: **0 174 571**
**A2**

# (12) EUROPÄISCHE PATENTANMELDUNG

(21) Anmeldenummer: **85110941.3**

(22) Anmeldetag: **30.08.85**

(51) Int. Cl.[4]: **C 07 K 5/06**
A 61 K 37/02

(30) Priorität: **03.09.84 DE 3432307**

(43) Veröffentlichungstag der Anmeldung:
**19.03.86 Patentblatt 86/12**

(84) Benannte Vertragsstaaten:
**AT BE CH DE FR GB IT LI LU NL SE**

(71) Anmelder: **BOEHRINGER INGELHEIM KG**

**D-6507 Ingelheim am Rhein(DE)**

(84) Benannte Vertragsstaaten:
**BE CH DE FR IT LI LU NL SE AT**

(71) Anmelder: **Boehringer Ingelheim International G.m.b.H**

**D-6507 Ingelheim am Rhein(DE)**

(84) Benannte Vertragsstaaten:
**GB**

(72) Erfinder: **Roos, Otto, Dr.**
**Elsheimer Strasse 36**
**D-6501 Schwabenheim(DE)**

(72) Erfinder: **Lösel, Walter, Dr.**
**Im Herzenacker 26**
**D-6535 Gau-Algesheim(DE)**

(72) Erfinder: **Schnorrenberg, Gerd, Dr.**
**Fichtenweg 13**
**D-6507 Ingelheim/Rhein(DE)**

(72) Erfinder: **Wiedemann, Ingrid, Dr.**
**Ernst-Ludwig-Strasse 61**
**D-6535 Gau-Algesheim(DE)**

(72) Erfinder: **Gaida, Wolfram, Dr.**
**Paul-Clemen-Strasse 14**
**D-6507 Ingelheim/Rhein(DE)**

(72) Erfinder: **Hoefke, Wolfgang, Dr.**
**Rosselstrasse 27**
**D-6200 Wiesbaden(DE)**

## (54) Aminosäurederivate, Verfahren zu ihrer Herstellung und pharmazeutische Zusammensetzungen.

(57) Die Erfindung betrifft neue Aminosäurederivate der allgemeinen Formel

$$R_1 - \underset{}{\overset{SR_2}{\overset{|}{CH}}} - CO - NH - \overset{CH_3}{\overset{|}{CH}} - CO - A \qquad (I)$$

sowie ihre Salze.

Gegenstand der Erfindung sind fernet Verfahren zu ihrer Herstellung, pharmazeutische Zusammensetzungen und die Anwendung als Arzneimittel.

In Formel I bedeuten:

$R_1$ H, Phenyl, Benzyl, Phenethyl;

$R_2$ H, Acetyl, Benzoyl, 3-Sulfonamido-4-chlor-benzoyl, 3-Sulfonamido-4-chlor-6-hydroxy-benzoyl, 3-Sulfonamido-4-chlor-5-[(furyl)-amino]-benzoyl, 2,3-Dichlor-4-(β-phenyl-acryloyl)-phenoxy-acetyl, Pivaloyl, $C_1$-$C_2$-Alkylaminocarbonyl, $C_1$-$C_2$-Alkylaminothiocarbonyl, Acyl oder den Rest

$$- S - \underset{R_1}{\underset{|}{CH}} - CO - NH - \overset{CH_3}{\overset{|}{CH}} - CO - A\ ;$$

A den Rest einer der α-Aminosäuren

$$\overset{R_3}{\overset{|}{HN}} \text{——} \overset{R_4}{\overset{\vdots}{C(H)}} \text{——} COR_5$$

oder

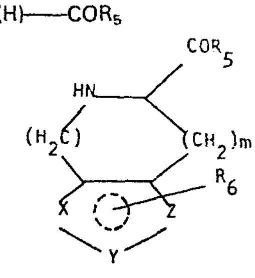

./...

EP 0 174 571 A2

$R_3$ Wasserstoff, $C_1$-$C_4$-Alkyl, gerade oder verzeigt, $C_5$-$C_7$-Cycloalkyl, wobei ein Phenylring ankondensiert sein kann, Phenyl, Phenyl-$C_1$-$C_4$-alkyl, einen Hetero-$C_1$-$C_4$-alkylrest, wobei der Heterocyclus aus einem 5- oder 6-Ring mit 1 - 2 der Heteroatome O, S oder N besteht,

$R_4$ Wasserstoff, $C_1$-$C_4$-Alkyl, gerade oder verzeigt, Phenyl, Phenyl-$C_1$-$C_4$-alkyl oder einen Hetero-$C_1$-$C_4$-alkylrest, wobei der Heterocyclus aus einem 5- oder 6-Ring mit 1 - 2 der Heteroatome O, S oder N besteht, oder

$R_3$ und $R_4$ zusammen bilden mit dem N- und dem C-Atom einen 5-, 6- oder 7-gliedrigen Ring, der gesättigt sein oder eine Doppelbindung enthalten kann, oder einen 4-, 5- oder 6-gliedrigen Ring, der ein oder zwei weitere der Heteroatome O, S oder N enthält;

$R_5$ OH, $C_1$-$C_4$-ω-Hydroxyalkoxy, $C_1$-$C_4$-Alkoxy, Phenyl-$C_1$-$C_4$-alkoxy, $C_1$-$C_4$-Alkylamino, $(C_1$-$C_4)_2$-Dialkylamino, eine der Gruppen

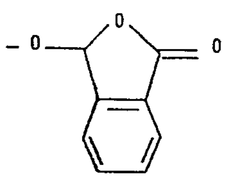

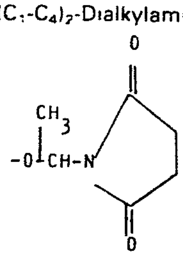

oder 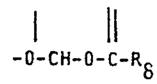

oder eine α-Aminosäure, die peptidartig mit der CO-Gruppe des Moleküls verknüpft ist;

$R_6$ $C_1$-$C_4$-Alkyl, $C_1$-$C_3$-Alkenyl, $C_1$-$C_3$-Alkinyl, gerade oder verzweigt, Hydroxy, Nitro, Amino, $C_1$-$C_4$-Alkoxy, Mercapto, $C_1$-$C_4$-Alkylthio, Hydroxy-$C_1$-$C_4$-alkyl, Mercapto-$C_1$-$C_4$-alkyl, F, Cl, Br, Amino-$C_1$-$C_4$-alkyl, Sulfonamido, Methylendioxy, Fluor-$C_1$-$C_4$-alkyl, Chlor-$C_1$-$C_4$-alkyl, Brom-$C_1$-$C_4$-alkyl, Cyano oder Trifluormethyl;

$R_7$ Wasserstoff oder Methyl;

$R_8$ $C_1$-$C_4$-Alkyl, gerade oder verzweigt, wobei der Alkylrest durch F, Cl, Br, $CF_3$, Phenyl oder Pyridyl substituiert sein kann;

X, Y und Z O, S, $CR_{10}$, $CHR_{10}$,

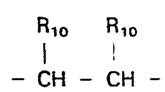 oder 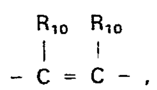 ,

mit der Maßgabe, daß nur einer der Reste X, Y und Z O, S

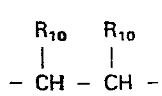 oder 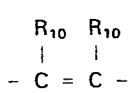 und

einer oder zwei Reste X, Y und Z $NR_9$ bedeuten können;

$R_9$ Wasserstoff oder $C_1$-$C_4$-Alkyl, gerade oder verzweigt;

$R_{10}$ Wasserstoff, oder zusammen mit einem vicinal stehenden Rest $R_{10}$ einen Phenylring oder, für m und n = 1, dessen Dihydroform mit der Doppelbindung in Konjugation zur C-terminalen Carboxygruppe und

m und n jeweils 0, 1 oder 2, wobei die Summe aus m und n 1 oder 2 ist.

Die neuen Verbindungen besitzen eine starke Angiotensin-I-Converting-Enzym-Hemmung und sollen als Hypotensiva Verwendung finden.

Die Erfindung betrifft neue Aminosäurederivate der allgemeinen Formel

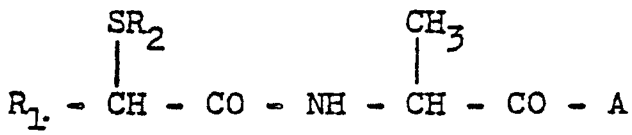 (I)

sowie ihre Salze.
Gegenstand der Erfindung sind ferner Verfahren zu ihrer Herstellung, pharmazeutische Zusammensetzungen und die Anwendung als Arzneimittel.

In Formel I bedeuten:

$R_1$ H, Phenyl, Benzyl, Phenethyl;
$R_2$ H, Acetyl, Benzoyl, 3-Sulfonamido-4-chlor-benzoyl, 3-Sulfonamido-4-chlor-6-hydroxy-benzoyl, 3-Sulfonamido-4-chlor-5-[(furyl)-amino]-benzoyl, 2,3-Dichlor-4-(ß-phenyl-acryloyl)-phenoxy-acetyl, Pivaloyl, $C_1$-$C_2$-Alkylaminocarbonyl, $C_1$-$C_2$-Alkylaminothiocarbonyl, Pivaloyloxymethyl oder den Rest

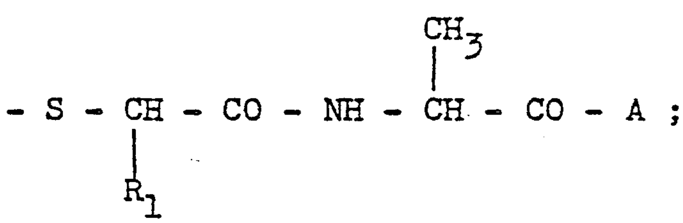

A den Rest einer der α-Aminosäuren

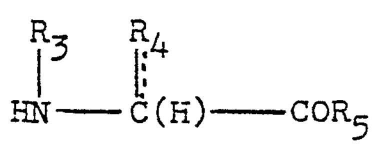

oder

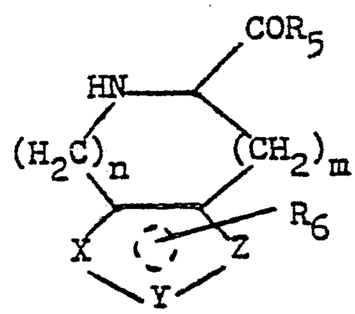

$R_3$ Wasserstoff, $C_1$-$C_4$-Alkyl, gerade oder verzweigt, $C_5$-$C_7$-Cycloalkyl, wobei ein Phenylring ankondensiert sein kann, Phenyl, Phenyl-$C_1$-$C_4$-alkyl, einen Hetero-$C_1$-$C_4$-alkylrest, wobei der Heterocyclus aus einem 5- oder 6-Ring mit 1 - 2 der Heteroatome O, S oder N besteht,

$R_4$ Wasserstoff, $C_1$-$C_4$-Alkyl, gerade oder verzweigt, Phenyl, Phenyl-$C_1$-$C_4$-alkyl oder einen Hetero-$C_1$-$C_4$-alkylrest, wobei der Heterocyclus aus einem 5- oder 6-Ring mit 1 - 2 der Heteroatome O, S oder N besteht,

$R_3$ und $R_4$ zusammen bilden mit dem N- und dem C-Atom einen 5-, 6- oder 7-gliedrigen Ring, der gesättigt sein oder eine Doppelbindung enthalten kann und der gegebenenfalls durch Oxo, Dimercaptoethylene oder ein oder zwei Hydroxy oder Methoxygruppe substituiert ist, oder einen 4-, 5- oder 6-gliedrigen Ring, der ein oder zwei weitere der Heteroatome O, S oder N enthält;

$R_5$ OH, $C_1$-$C_4$-$\omega$-Hydroxyalkoxy, $C_1$-$C_4$-Alkoxy, Phenyl-$C_1$-$C_4$-alkoxy, $C_1$-$C_4$-Alkylamino, Di-($C_1$-$C_4$-alkyl)-amino, eine der Gruppen

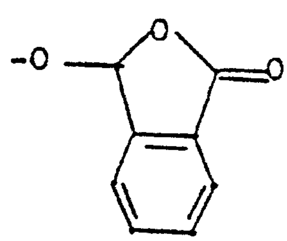

,

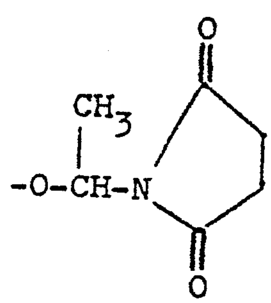

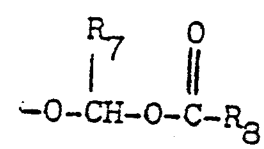

oder

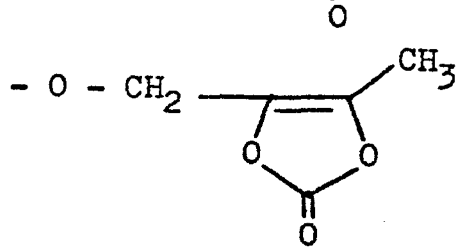

oder eine α-Aminosäure, die peptidartig mit der CO-Gruppe des Moleküls verknüpft ist;

$R_6$ $C_1$-$C_4$-Alkyl, $C_1$-$C_3$-Alkenyl, $C_1$-$C_3$-Alkinyl, gerade oder verzweigt, Hydroxy, Nitro, Amino, $C_1$-$C_4$-Alkoxy, Mercapto, $C_1$-$C_4$-Alkylthio, Hydroxy-$C_1$-$C_4$-alkyl, Mercapto-$C_1$-$C_4$-alkyl, F, Cl, Br, Amino-$C_1$-$C_4$-alkyl, Sulfonamido, Methylendioxy, Fluor-$C_1$-$C_4$-alkyl, Chlor-$C_1$-$C_4$-alkyl, Brom-$C_1$-$C_4$-alkyl, Cyano oder Trifluormethyl;

$R_7$ Wasserstoff oder Methyl;

$R_8$ $C_1$-$C_4$-Alkyl, gerade oder verzweigt, wobei der Alkylrest durch F, Cl, Br, $CF_3$, Phenyl oder Pyridyl substituiert sein kann;

X, Y und Z O, S, $CR_{10}$, $CHR_{10}$,

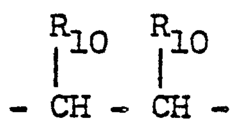 oder 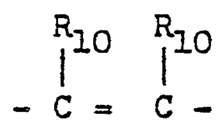 ,

mit der Maßgabe, daß nur einer der Reste X, Y und Z O, S

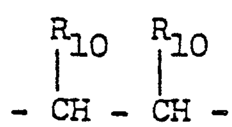 oder 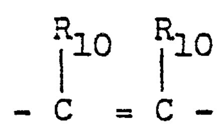 und

einer oder zwei der Reste X, Y und Z $NR_9$ bedeuten können;

$R_9$ Wasserstoff oder $C_1$-$C_4$-Alkyl, gerade oder verzweigt;

$R_{10}$ Wasserstoff, oder zusammen mit einem vicinal stehenden Rest $R_{10}$ einen Phenylring oder, für m und n = 1, dessen Dihydroform mit der Doppelbindung in Konjugation zur C-terminalen Carboxygruppe und

m und n jeweils 0,1 oder 2, wobei die Summe aus m und n 1 oder 2 ist.

Bedeuten die Reste $R_3$ und/oder $R_4$ einen durch einen 5- oder 6-gliedrigen Heterocyclus substituierten $C_1$-$C_4$-Alkylrest (im Text abgekürzt als "Hetero-$C_1$-$C_4$-alkylrest" bezeichnet), so seien als Beispiele für den Heterocyclus genannt Thiophen, Furan, Pyrrol, Thiazol, Oxazol, Isoxazol, Pyrazol, Imidazol, Pyran, Thiopyran, Pyridin, Morpholin, Pyrazin, Pyrimidin, Pyridazin, Oxathiin sowie deren Di- und Tetrahydroformen.

Bilden die Reste $R_3$ und $R_4$ zusammen mit dem N- und C-Atom einen 5-Ring so werden als Beispiel für den unsubstituierten Ring Pyrrolidindiyl und für den subsituierten Ring die Gruppe

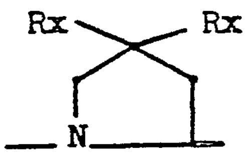

wobei Rx und Rx' die gleich oder verschieden sein können und Wasserstoff, Hydroxy oder Methoxy bedeuten, oder Rx und Rx' zusammen Oxo oder Dimercaptoethylen bedeuten.

Die als Bedeutung für den Rest $R_5$ genannten, gegebenenfalls substituierten Alkylgruppen können gerade oder verzweigt sein; als α-Aminosäuren, die peptidartig mit der CO-Gruppe des Moleküls verbunden sein können, kommen insbesondere in Betracht Prolin, Valin, Leucin, Isoleucin und Phenylalanin.

Bei einer der Bedeutungen für A können die an die Pyrrolidin- beziehungsweise Piperidincarbonsäure ankondensierten 5- oder 6-Ring-Heterocyclen gesättigt oder ungesättigt sein. Bevorzugte Heterocyclen sind Furan, Pyrrol, Thiophen, Benzofuran, Indol, Benzothiophen, Oxazol, Imidazol, Thiazol, Isoxazol, Pyrazol, Pyrrolidin, Tetrahydrofuran, Tetrahydrothiophen, Pyridin, Pyridazin, Chinolin, Isochinolin oder Piperidin.

Die neuen Verbindungen weisen im allgemeinen mehrere Asymmetriezentren auf und liegen daher als Diastereomere oder in Form ihrer Racemate beziehungsweise ihrer racemischen Gemische vor. Die Erfindung umfaßt sowohl die racemischen Gemische als auch die einzelnen Diastereomeren. Bevorzugt sind diejenigen Enantiomeren, bei denen die asymmetrischen C-Atome in der S-Konfiguration vorliegen.
Die erwähnten Racemate können in üblicher Weise, z.B. durch fraktionierte Kristallisation, durch chemische oder durch biochemische Spaltung, in ihre sterisch einheitlichen Formen angereichert oder rein erhalten werden.

Die erfindungsgemäßen Säuren bilden Salze mit anorganischen und organischen Basen. Beispiele für anorganische Basen sind Ammoniak, Alkalimetallhydroxyde wie Natrium- oder Kaliumhydroxyd oder Erdalkalimetallhydroxyde wie Calcium- oder Magnesiumhydroxyd; an organischen Basen seien genannt Dicyclohexylamin, N,N'-Dibenzyläthylendiamin, N,N'-Bis-(dehydroabietyl)-äthylendiamin, N-Methyl-D-glycamin, Arginin oder Lysin.

Die neuen Stoffe der allgemeinen Formel I können nach verschiedenen Verfahren erhalten werden:

a) Durch Kondensation einer Säure der allgemeinen Formel

$$R_1 - \underset{}{\overset{SR_2}{\overset{|}{CH}}} - COOH \qquad (II)$$

worin
$R_1$ und $R_2$ die oben angegebene Bedeutung besitzen, mit einem Dipeptid der allgemeinen Formel

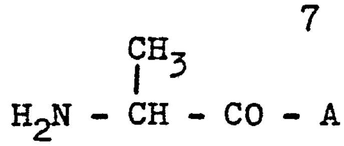

(III)

worin

A die oben angegebene Bedeutung besitzt.

b) Durch Kondensation einer Verbindung der allgemeinen Formel

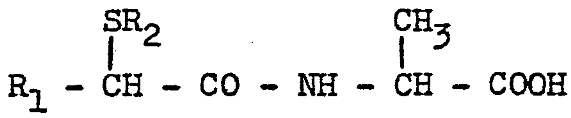

(IIa)

worin

$R_1$ und $R_2$ die oben angegebene Bedeutung haben, mit einer Aminosäure A, wobei A die oben angegebene Bedeutung besitzt.

Eine Verbindung der allgemeinen Formel IIa kann durch peptidische Verknüpfung einer Verbindung der allgemeinen Formel II mit Alanin erhalten werden.

c) Durch Umsetzung einer Verbindung der allgemeinen Formel

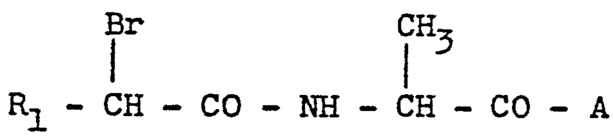

(V)

worin

$R_1$ und A die oben angegebene Bedeutung besitzen, mit einer Mercaptoverbindung der allgemeinen Formel

$$HS - R_2 \qquad (VI)$$

worin $R_2$ die oben angeführte Bedeutung besitzt.

Verbindungen gemäß Formel V. können durch Kondensation einer Bromcarbonsäure der allgemeinen Formel

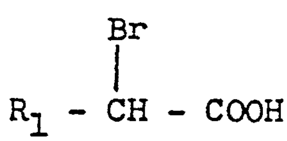

(IV)

worin

$R_1$ die oben angeführte Bedeutung besitzt, mit einem Dipeptid der allgemeinen Formel II erhalten werden

d) Kondensation einer Verbindung der allgemeinen Formel

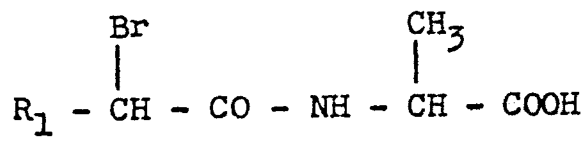

(IVa)

worin
$R_1$ die oben angeführte Bedeutung besitzt, mit einer Aminosäure A, wobei A die vorstehend angegebene Bedeutung besitzt und anschließender Umsetzung des so erhaltenen Kondensationsproduktes der allgemeinen Formel V mit einer Mercaptoverbindung der allgemeinen Formel VI.

Eine Verbindung der allgemeinen Formel IVa kann durch peptidartige Verknüpfung einer Verbindung der allgemeinen Formel IV mit Alanin erhalten werden.

e) Zur Herstellung von Verbindungen der Formel I, in denen $R_2$ Wasserstoff bedeutet:
Verseifung einer Verbindung der Formel I, in der $R_2$ $C_1$-$C_4$-Acyl, vor allem Acetyl oder Propionyl, oder Benzoyl bedeutet, mit basischen Mitteln, insbesondere wäßrigen Alkalien.

f) Zur Herstellung solcher Verbindungen der Formel I, in denen $R_2$ die obigen Bedeutungen hat, ausgenommen Wasserstoff:
Umsetzung von Verbindungen der Formel I, in denen $R_2$ Wasserstoff bedeutet, mit Verbindungen der Formel $B-R_2$, worin $R_2$ die obige Bedeutung, ausgenommen Wasserstoff, hat und B für eine leicht abspaltbare Gruppe, z.B. Chlor, Brom, Tosyl, Mesyl, steht, vorzugsweise in Gegenwart eines säurebindenden Mittels, etwa Alkalicarbonat, Alkalibasen.

In den obigen Verfahren a) bis f) wird jede Schutzgruppe auf einer geeigneten Stufe des Verfahrens abgespalten. Man überführt gegebenenfalls ein so erhaltenes Endprodukt der allgemeinen Formel in ein Salz oder wenn $R_5$ -OH ist in ein Ester gemäß Formel I.

Die vorstehend beschriebenen Kondensationen erfolgen nach allgemein üblichen Methoden, wie sie in Houben-Weyl, Band XV/1, 4. Auflage, 1974 für die Synthese von Peptiden beschrieben worden sind.
Im allgemeinen benutzt man die in der Peptidchemie üblichen

Kupplungsreaktionen unter Verwendung einer aktivierten Form der Säuren II, IIa, IV und IVa; als carboxylaktivierende Gruppen kommen in Frage Säurechloride, Azide, Anhydride, p-Nitrophenylester, Trichlorphenylester, Thiophenylester, o-Cyanomethylester usw.. Als Kupplungsmittel werden bevorzugt Carbonyldiimidazol, Dicyclohexylcarbodiimid, Äthoxyacetylen, Diphenylphosphorylazid oder Chlorameisensäureester; die Umsetzung erfolgt bei Temperaturen zwischen 0°C und + 10°C.

Im vorliegenden Fall hat sich die Verwendung von Methylenchlorid als Lösungsmittel zusammen mit Dicyclohexylcarbodiimid und Triäthylamin als vorteilhaft erwiesen.

Die Umsetzung der Brom-Zwischenprodukte mit einer Mercaptoverbindung der allgemeinen Formel VI erfolgt vorteilhaft in Äther als Lösungsmittel. Durch die Umsetzung mit Thiolsäuren (z.B. Thiolessigsäure) werden die entsprechenden Acylmercaptoverbindungen erhalten; die Acylgruppe kann mit starken Basen abgespalten werden.

Dimere der allgemeinen Formel I ($R_2$ =

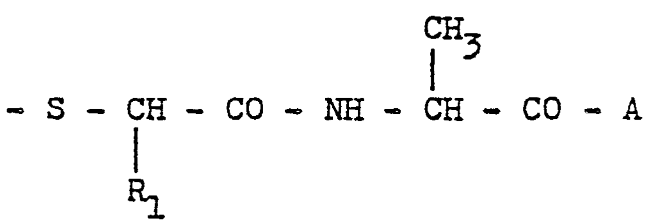

)

erhält man beispielsweise

α) durch Oxydation einer Verbindung der allgemeinen Formel I, in der $R_2$ Wasserstoff bedeutet, mit einem Oxydationsmittel wie Sauerstoff oder Jod;

ß) durch Umsetzung einer Verbindung der allgemeinen Formel IVa mit Thiolessigsäure zu einer Verbindung IIa und anschließender Verseifung zu einer Verbindung mit freier Mercaptogruppe. Die Oxydation zum entsprechenden Dimeren erfolgt wie unter α) beschrieben; das erhaltene Zwischenprodukt wird, wie unter Verfahren b) auf Seite 7 beschrieben, mit einer Aminosäure A kondensiert.

Bevorzugt sind solche Verbindungen der allgemeinen Formel I, in denen $R_1$ Wasserstoff, Phenyl, Benzyl oder Phenethyl; $R_2$ Wasserstoff, Acetyl oder 3-Sulfonamido-4-chlor-benzoyl und A Prolin bedeutet.

Nach den oben beschriebenen Verfahren können die folgenden Endprodukte, gegebenenfalls in Form ihrer Salze, erhalten werden:

1. N-[N-(2-Mercaptoacetyl)-alanyl]-prolin,
2. N-[N-(2-Phenyl-2-mercaptoacetyl)-alanyl]-prolin,
3. N-[N-(3-Phenyl-2-mercaptopropanoyl)-alanyl]-prolin,
4. N-[N-(4-Phenyl-2-mercaptobutyryl)-alanyl]-prolin,
5. N-[N-(2-Acetylmercaptoacetyl)-alanyl]-prolin,
6. N-[N-(2-Phenyl-2-acetylmercaptoacetyl)-alanyl]-prolin,
7. N-[N-(3-Phenyl-2-acetylmercaptopropanoyl)-alanyl]-prolin,
8. N,N'-[N,N'-{2,2'-dithiobis-(3-phenylpropanoyl)}-bis-S-alanyl]-bis-S-prolin,
9. N-[N-(3-Phenyl-2-S-mercaptopropanoyl)-S-alanyl]-S-prolin-pivaloyloxymethylester,
10. N-[N-(3-Phenyl-2-S-{4-chlor-3-sulfon-amindobenzoyl}-mercaptopropanoyl)-S-alanyl]-S-prolin,
11. N-[N-(3-Phenyl-2-S-mercaptopropanoyl)-S-alanyl]-S-prolyl-S-phenylalanin,

12. N-[N-(3-Phenyl-2-S-mercaptopropanoyl)-S-alanyl]-S-prolyl-S-valin,

13. N-[N-(3-Phenyl-2-S-mercaptopropanoyl)-S-alanyl]-S-prolyl-S-prolin,

14. N-[N-(3-Phenyl-2-S-mercaptopropanoyl)-S-alanyl]-S-thiazolidin-4-carbonsäure,

15. N-[N-(3-Phenyl-2-S-mercaptopropanoyl)-S-alanyl]-4,5,6,7-tetrahydro-thieno[2,3-c]pyridin-7-carbonsäure,

16. N-[N-(3-Phenyl-2-S-mercaptopropanoyl)-S-alanyl]-4,5,6,7-tetrahydro-thieno[3,2-c]pyridin-4-carbonsäure,

17. N-[N-(3-Phenyl-2-S-benzoylmercaptopropanoyl)-S-alanyl]-S-prolin,

18. N-[N-(3-Phenyl-2-S-pivaloylmercaptopropanoyl)-S-alanyl]-S-prolin,

19. N-[N-(3-Phenyl-2-S-acetylmercaptopropanoyl)-S-alanyl]-S-prolin-pivaloyloxymethylester,

20. N-[N-(3-Phenyl-2-S-benzoylmercaptopropanoyl)-S-alanyl]-S-prolin-pivaloyloxymethylester,

21. N-[N-(3-Phenyl-2-S-pivaloylmercaptopropanoyl)-S-alanyl]-S-prolin-pivaloyloxymethylester,

22. N-[N-(3-Phenyl-2-S-acetylmercaptopropanoyl)-S-alanyl]-N-cyclopentylglycin,

23. N-[N-(3-Phenyl-2-S-methylaminothiocarbonyl-mercaptopropanoyl)-S-alanyl]-S-prolin,

24. N-[N-(3-Phenyl-2-S-ethylaminocarbonyl-mercaptopropanoyl)-S-alanyl]-S-prolin.

Bei den Ausgangsstoffen handelt es sich meist um bekannte Verbindungen, auch eventuell neue Ausgangsstoffe werden nach an sich bekannten Verfahren hergestellt; Verbindungen der allgemeinen Formel III erhält man z.B. durch peptidartige Verknüpfung von Alanin mit einer Aminosäure A.

Die Aminosäurederivate der allgemeinen Formel I besitzen eine starke, lang anhaltende blutdrucksenkende Wirkung. Diese beruht auf einer Hemmung des Angiotensin I Converting Enzyms und damit einer Blockierung der Bildung des Vasokonstriktors Angiotensin II aus Angiotensin I. Darüber hinaus wirken die neuen Verbindungen hemmend auf das für den Bradykininabbau verantwortliche Enzym Kininase II, das als identisch mit dem oben genannten Converting Enzym gilt. Da Bradykinin eine gefäßerweiternde Wirkung besitzt, wird der blutdrucksenkende Effekt durch diese zusätzliche Wirkung verstärkt. Die durch Bradykinin erzeugte Blutdrucksenkung an normalen Ratten wird durch die neuen Verbindungen verstärkt. Ausdruck dieser Wirkung könnte auch die an unvorbehandelten genetischen Hochdruckratten beobachtete blutdrucksenkende Wirkung sein.

So erzeugt das N-[N-(3-Phenyl-2-S-mercaptopropanoyl)-S-alanyl]-S-prolin an der narkotisierten Ratte in einer Dosierung von 0,3 mg/kg i.v. eine lang anhaltende Blutdrucksenkung von 67 %, wenn man vorher durch eine i.v.-Gabe von 0,1 mg/kg Angiotensin I den Blutdruck steigert.

An der wachen Ratte wird nach einer Stimulation mit 0,2 mg/kg Angiotensin I (i.v.) eine Blutdrucksteigerung bewirkt, die mit 1 mg/kg p.o. der oben genannten Verbindung lange anhaltend zu loo % aufgehoben wird.

Am Kaninchen (Blutdruckversuch aus der Carotis) wird nach Gabe von 1 mg/kg der Substanz, i.v., eine Blutdrucksenkung von 37 mm beobachtet.

Das N-[N-(3-Phenyl-2-S-mercaptopropanoyl)-S-alanyl]-S-prolin zeigt am Angiotensin I Converting Enzym in vitro eine Hemmaktivität $IC_{50} = 5,0 \cdot 10^{-9}$ [M].
Das S-Acetylanaloge der Verbindung weist einen Hemmwert $IC_{50} = 7,4 \cdot 10^{-9}$ [M] auf.
Als $IC_{50}$ wird hierbei diejenige Hemmstoffkonzentration bezeichnet, bei der das Angiotensin I Converting Enzym zu 50 % gehemmt wird (s. H. S. Cheung, D. W. Cushman, Biochem. Biophys. Acta 293, 451 (1973)).

Für die Anwendung in der Therapie werden die neuen Verbindungen mit üblichen pharmazeutischen Füll- oder Trägerstoffen, Streck-, Spreng-, Binde-, Gleit-, Dickungs- oder Verdünnungsmitteln gemischt.
Als pharmazeutische Zubereitungsformen kommen zum Beispiel Tabletten, Kapseln, Zäpfchen, Lösungen, Säfte, Emulsionen oder dispersible Pulver in Frage, wobei gewünschtenfalls weitere bekannte Wirkstoffe, z.B. Saluretica, Diuretica und/oder Antihypertonica zugefügt werden können.

Entsprechende Tabletten können beispielsweise durch Mischen des oder der Wirkstoffe mit bekannten Hilfsstoffen, beispielsweise inerten Verdünnungsmitteln, wie Calciumcarbonat, Calciumphosphat oder Milchzucker, Sprengmitteln, wie Maisstärke oder Alginsäure, Bindemitteln, wie Stärke oder Gelatine, Schmiermitteln, wie Magnesiumstearat oder Talk, und/oder Mitteln zur Erzielung des Depoteffektes, wie Carboxypolymethylen, Carboxymethylcellulose, Celluloseacetatphthalat, oder Polyvinylacetat erhalten werden. Die Tabletten können auch aus mehreren Schichten bestehen.

Entsprechend können Dragees durch Überziehen von analog den Tabletten hergestellten Kernen mit üblicherweise in Drageeüberzügen verwendeten Mitteln, beispielsweise Kollidon oder Schellack, Gummi arabicum, Talk, Titandioxid oder Zucker, hergestellt werden. Zur Erzielung eines Depoteffektes oder zur Vermeidung von Inkompatibilitäten kann der Kern auch aus mehreren Schichten bestehen. Desgleichen kann auch die Drageehülle zur Erzielung eines Depoteffektes aus mehreren Schichten bestehen, wobei die oben bei den Tabletten erwähnten Hilfsstoffe verwendet werden können.

Säfte der erfindungsgemäßen Wirkstoffe beziehungsweise Wirkstoffkombinationen können zusätzlich noch ein Süßungsmittel, wie Saccharin, Cyclamat, Glycerin oder Zucker sowie ein geschmacksverbesserndes Mittel, z.B. Aromastoffe, wie Vanillin oder Orangenextrakt, enthalten. Sie können außerdem Suspendierhilfsstoffe oder Dickungsmittel, wie Natriumcarboxymethylcellulose, Netzmittel, beispielsweise Kondensationsprodukte von Fettalkoholen mit Äthylenoxid, oder Schutzstoffe, wie p-Hydroxybenzoate, enthalten.

Injektionslösungen werden in üblicher Weise, z.B. unter Zusatz von Konservierungsmitteln, wie p-Hydroxybenzoaten, oder Stabilisatoren, wie Alkalisalzen der Äthylendiamintetraessigsäure und unter Zugabe geeigneter Lösungsvermittler hergestellt und in Injektionsflaschen oder Ampullen abgefüllt.

Die einen oder mehrere Wirkstoffe beziehungsweise Wirkstoffkombinationen enthaltenden Kapseln können beispielsweise hergestellt werden, indem man die Wirkstoffe mit inerten Trägern, wie Milchzucker oder Sorbit, mischt und in Gelatinekapseln einkapselt.

Die tägliche Dosis für die Anwendung der erfindungsgemäßen Verbindungen der allgemeinen Formel I beziehungsweise ihre Salze soll 5 - 500 vorzugsweise 10 - 100 mg betragen und kann in 1 - 4 Einzeldosen verabreicht werden.

Die folgenden Beispiele dienen zur näheren Erläuterung der Erfindung. Die Struktur aller synthetisierter Beispiele wurde durch NMR-Spektren bestätigt.

Beispiel 1

N-[N-(3-Phenyl-2-S-mercaptopropanoyl)-S-alanyl]-S-prolin

Eine Lösung von 5,33 g (70 mMol) Thiolessigsäure in 150 ml wasserfreiem Ether wird unter Stickstoff und Eiskühlung langsam mit 7,1 g (70 mMol) Triethylamin versetzt und anschließend tropfenweise zu einer Lösung von 15,9 g (35 mMol) N-[N-(3-Phenyl-2-R-brompropanoyl)-S-alanyl]-S-prolin-tert.butylester in 75 ml wasserfreiem Ether unter Rühren gegeben. Man kocht danach noch 90 Minuten am Rückfluß, filtriert die vorher gekühlte Lösung und wäscht mit verdünnter $KHSO_4$- und $NaHCO_3$-Lösung, Wasser und gesättigter NaCl-Lösung. Nach dem Trocknen über $MgSO_4$ wird das Lösungsmittel abdestilliert und der ölige Rückstand (16,4 g) über Kieselgel chromatographiert (Laufmittel: Essigester, n-Hexan (2:1)). 13,1 g (= 83,4 % d.Th.)des Acetylmercapto-tert.Butylesters werden als farbloses, zähes Öl erhalten;
Rf = 0,41 (Essigester, n-Hexan = 2:1, Silicagel).

Zur Verseifung des tert.-Butylesters wird eine Lösung von 13,1 g (29,2 mMol) des obigen Esters in 65 ml Anisol und 130 ml Trifluoressigsäure 2 Stunden bei Raumtemperatur gerührt. Anschließend wird im Vakuum eingeengt und der Rückstand jeweils dreimal in ca. 100 ml Aceton, dann in Chloroform, gelöst und nachfolgend im Vakuum einrotiert. Der Rückstand wird in Dichlormethan gelöst und zweimal mit gesättigter $NaHCO_3$-Lösung extrahiert. Die vereinigten $NaHCO_3$-Lösungen werden mit Dichlormethan gewaschen, die wäßrige Phase mit konz. HCl angesäuert und mit Dichlormethan extrahiert. Die Dichlormethanlösung wird mit Wasser, gesättigter NaCl-Lösung gewaschen, über $MgSO_4$ getrocknet und im Vakuum eingedampft.

10,6 g (= 92,5 % d.Th.) der freien Säure, N-[N-(3-Phenyl-2-S-acetylmercaptopropanoyl)-S-alanyl]-S-prolin, werden als erstarrter Schaum isoliert;
Rf = 0,6 (Chloroform, Methanol, Eisessig = 90:10:5, Silicagel).

Zur Verseifung des Thiolessigsäureesters werden zu 10,6 g (27 mMol) N-[N-(3-Phenyl-2-S-acetylmercapto-propanoyl)-S-alanyl]-S-prolin unter $N_2$-Atmosphäre und Rühren 250 ml $H_2O$ und 22 ml 5 n NaOH gegeben und die Lösung 1,5 Stunden bei Raumtemperatur belassen. Danach werden weitere 10 ml 5 n NaOH gegeben und bei der gleichen Temperatur 1,5 Stunden lang weitergerührt. Die wäßrige Lösung wird zweimal mit Dichlormethan gewaschen, mit halbkonzentrierter HCl angesäuert und dreimal mit Dichlormethan extrahiert. Die organische Phase wird mit gesättigter NaCl-Lösung gewaschen, über $MgSO_4$ getrocknet und eingedampft.
9,2 g (= 97,2 % d.Th.) der Titelverbindung werden als erstarrter Schaum isoliert, deren Struktur aufgrund der IR- und NMR-Daten bestätigt wurde.
Rf = 0,6 (Chloroform, Methanol, Eisessig = 90:10:5, Silicagel).

Die Ausgangsverbindungen werden wie folgt hergestellt:

<u>3-Phenyl-2-R-brompropionsäure</u>

Lit.: Chemistry of the Amino Acids, Vol. I, N.Y. (1961), p. 165; Ann. 357, 1 (1907).

Abgewandelte Literaturvorschrift:
51,2 g (0,31 Mol) R-Phenylalanin werden nacheinander unter Rühren mit 620 ml 2,5 n $H_2SO_4$ und 125 g KBr (1,05 Mol) versetzt. Nach Abkühlen der Lösung auf 0°C werden portionsweise innerhalb von 80 Minuten

32,75 g (0,475 Mol) $NaNO_2$ gegeben und 1 Stunde bei Raumtemperatur weitergerührt. Die entstandene Emulsion wird dreimal mit je ca. 200 ml Ether extrahiert, die organische Phase mit Wasser und gesättigter NaCl-Lösung gewaschen, über $MgSO_4$ getrocknet und der Ether abdestilliert. Der ölige Rückstand (63 g) wird über Kieselgel mit Essigester, n-Hexan (= 2:1) als Elutionsmittel chromatographisch gereinigt.
45,1 g der Titelverbindung werden als rötliches Öl erhalten, die Struktur und die optische Reinheit NMR-spektroskopisch bestätigt. Die Herstellung der S- und R,S-Analogen erfolgt nach der gleichen Vorschrift.

N-(3-Phenyl-2-R-brompropanoyl)-S-alanin

Zu einer Lösung von 22,9 g (0,1 Mol) 3-Phenyl-2-R-brompropionsäure, 13,9 g (0,1 Mol) S-Alaninmethylesterhydrochlorid und 10 g (0,1 Mol) Triethylamin in 500 ml wasserfreiem Dichlormethan werden unter Rühren bei 0°C 20,6 g (0,1 Mol) N,N'-Dicyclohexylcarbodiimid gegeben, 15 Minuten bei 0°C, dann über Nacht bei Raumtemperatur, weitergerührt. Nach Absaugen und Abdestillieren des Lösungsmittels wird der Rückstand in Essigester gelöst und nach Kühlung abfiltriert. Die Essigesterlösung wird nacheinander mit verdünnter $KHSO_4$-, gesättigter $NaHCO_3$-Lösung, Wasser, gesättigter NaCl-Lösung gewaschen und über $MgSO_4$ getrocknet. Nach Abdestillieren des Essigesters wird der ölige Rückstand (29,9 g) über Kieselgel chromatographiert (n-Hexan, Essigester = 2:1) und dabei 23,9 g (= 76 % d.Th.; entspr. 76 mMol) der Titelverbindung als Methylester erhalten.

Zu seiner Verseifung löst man in 150 ml Methanol, kühlt auf 0°C und setzt unter Rühren 80 ml 1 n NaOH (80 mMol) zu. Nach dreistündigem Rühren bei Raumtemperatur

wird im Vakuum eingedampft, der Rückstand mit Wasser verdünnt und dreimal mit Dichlormethan extrahiert. Nach Abziehen des in der Wasserphase gelösten Dichlormethans im Vakuum wird mit konzentrierter HCl angesäuert und die ausgefallenen Kristalle abfiltriert. Ausbeute: 17,8 g (= 78 % d.Th.) der Titelverbindung vom Fp. 156 - 157°C werden erhalten.
Rf = 0,7 (Chloroform, Methanol, Eisessig 90:10:5, Silicagel).

Analog werden das S S- und (R,S) S-Diastereomere erhalten.
Ein NMR-spektroskopischer Vergleich der S S- und R,S-Diastereomeren zeigt den stereoselektiven Verlauf der Synthese.

N-[N-(3-Phenyl-2-R-brompropanoyl)-S-alanyl]-S-prolin-tert.butylester

a) 17,8 g (59,3 mMol) N-(3-Phenyl-2-R-brompropanoyl)-S-alanin und 10,1 g (59,3 mMol) S-Prolin-tert.butylester werden in 250 ml wasserfreiem Dichlormethan gelöst und unter Rühren und Kühlen (0°C) mit 12,2 g (59,3 mMol) N,N'-Dicyclohexylcarbodiimid versetzt. Nach Absaugen des Dicyclohexylcarbodiimids wird das Lösungsmittel im Vakuum abdestilliert, der ölige Rückstand in Essigester gelöst und einige Zeit kaltgestellt. Nach erneutem Absaugen wird nacheinander mit verdünnter $KHSO_4$-Lösung, gesättigter $NaHCO_3$-Lösung, Wasser und gesättigter NaCl-Lösung gewaschen und nach Trocknen über $MgSO_4$ das Lösungsmittel im Vakuum abdestilliert. Der ölige Rückstand (24,2 g) wird über Kieselgel chromatographiert (Laufmittel: Essigester, n-Hexan (2:1)).

0174571

15,9 g (= 59,1 % d.Th.) der Titelverbindung können als farbloses, zähes Öl isoliert werden, das NMR-spektroskopisch und chromatographisch einheitlich erscheint.
Rf = 0,45 (Essigester, Hexan (2:1), Silicagel).

Analog werden die S S S- und R,S S S-analogen Diastereomeren erhalten.

b) Eine Lösung von 2,29 g (10 mMol) 3-Phenyl-2-S-brompropionsäure, 2,78 g (10 mMol) S-Alanyl-S-prolin-tert.butylester-Hydrochlorid und 1 g (10 mMol) Triethylamin werden in 100 ml wasserfreiem Dichlormethan gelöst und unter Rühren und Kühlen (0°C) mit 2,06 g (10 mMol) N,N'-Dicyclohexylcarbodiimid versetzt. Man rührt über Nacht bei Raumtemperatur weiter, kühlt und saugt vom ausgefallenen Dicyclohexylharnstoff ab. Das Lösungsmittel wird im Vakuum abdestilliert, der ölige Rückstand in Essigester gelöst, gekühlt, vom Ungelösten abfiltriert und nacheinander mit verdünnter $KHSO_4$-Lösung, gesättigter $NaHCO_3$-Lösung, Wasser und gesättigter NaCl-Lösung gewaschen. Nach Trocknen über $MgSO_4$ wird das Lösungsmittel abdestilliert und 4,3 g (= 94,8 % d.Th.) eines farblosen, zähen Öls erhalten.

Analog werden die R S S- und R,S S S-analogen Diastereomeren erhalten.

Beispiel 2

N-[N-(3-Phenyl-2-R-mercaptopropanoyl)-S-alanyl]-S-prolin

Analog Beispiel 1 erhält man beim Einsatz von N-[N-(3-Phenyl-2-S-brompropanoyl)-S-alanyl]-S-prolin-tert.butylester die diastereomere Titelverbindung N-[N-(3-Phenyl-2-R-mercaptopropanoyl)-S-alanyl]-S-prolin. Rf = 0,54 (Chloroform, Methanol, Eisessig = 90:10:5, Silicagel).

Beispiel 3

N-[N-(3-Phenyl-2-R,S-mercaptopropanoyl)-S-alanyl]-S-prolin

Analog Beispiel 1 erhält man beim Einsatz von N-[N-(3-Phenyl-2-R,S-brompropanoyl)-S-alanyl]-S-prolin-tert.butylester die Titelverbindung N-[N-(3-Phenyl-2-R,S-mercaptopropanoyl)-S-alanyl]-S-prolin.

Beispiel 4

N-[N-(Mercaptoacetyl)-S-alanyl]-S-prolin

Analog Beispiel 1 erhält man beim Einsatz von N-[N-Bromacetyl)-S-alanyl]-S-prolin-tert.butylester die Titelverbindung N-[N-(Mercaptoacetyl)-S-alanyl-S-prolin.
Rf = 0,35 (Chloroform, Methanol, Eisessig = 90:10:5, Silicagel).

Beispiel 5

N-[N-(2-Phenyl-2-R,S-mercaptoacetyl)-S-alanin]-S-prolin

Analog Beispiel 1 erhält man beim Einsatz von N-[N-(2-Phenyl-2-R,S-bromacetyl)-S-alanin]-S-prolin-tert.butylester die Titelverbindung N-[N-(2-Phenyl-2-R,S-mercaptoacetyl)-S-alanin]-S-prolin.
Rf = 0,48 (Chloroform, Methanol, Eisessig = 90:10:5, Silicagel).

Beispiel 6

N-[N-(4-Phenyl-2-R,S-mercaptobutanoyl)-S-alanin]-S-prolin

Analog Beispiel 1 erhält man beim Einsatz von N-[N-(4-Phenyl-2-R,S-brombutanoyl)-S-alanin]-S-prolin die Titelverbindung N-[N-(4-Phenyl-2-R,S-mercaptobutanoyl)-S-alanin]-S-prolin.
Rf = 0,59 (Chloroform, Methanol, Eisessig = 90:10:5, Silicagel).

Beispiel 7

N,N'-[N,N'-{2,2'-dithiobis-(3-phenylpropanoyl)}-bis-S-alanyl]-bis-S-prolin

2,5 g (4,95 mMol)N,N'-[2,2'-S-didthiobis-(3-phenylpropanoyl)]-bis-S-alanin und 1,69 g (9,9 mMol) S-Prolin-tert.butylester werden in 50 ml wasserfreiem Dichlormethan gelöst und unter Rühren und Eiswasserkühlung mit 2,0 g (9,90 mMol) N,N'-Dicyclohexylcarbodiimid versetzt. Es wird noch 15 Minuten, dann über Nacht bei Raumtemperatur nachgerührt, abgesaugt und vom Lösungsmittel abdestilliert. Der Rückstand wird in Essigester gelöst,

kaltgestellt, erneut abfiltriert und mit verdünnter $KHSO_4$-Lösung, gesättigter $NaHCO_3$-Lösung, Wasser und gesättigter NaCl-Lösung gewaschen. Nach Trocknen über $MgSO_4$ wird der Essigester im Vakuum abdestilliert und dabei 3,4 g eines erstarrten Schaumes erhalten.
Der Rückstand wird über Kieselgel chromatographiert (Laufmittel: Dichlormethan, Methanol (95:5)).
2,6 g der Titelverbindung in der Bis-tert.butylesterform chromatographisch und NMR-spektroskopisch einheitlich werden erhalten.

Zur Verseifung des Diesters wird eine Mischung von 2,6 g (3,2 mMol) des obigen dimeren tert.Butylesters, 13 ml Anisol und 26 ml Trifluoressigsäure 2 Stunden lang bei Raumtemperatur gerührt und anschließend im Vakuum zur Trockne eingedampft. Der Rückstand (2,7 g) wird jeweils dreimal mit Aceton und Chloroform versetzt und erneut im Vakuum abdestilliert. Das Rohprodukt wird in Dichlormethan aufgenommen, mit gesättigter $NaHCO_3$-Lösung gewaschen, die wäßrige Phase mit konzentrierter HCl angesäuert und der kristalline Niederschlag abgesaugt, mit Wasser gewaschen und getrocknet.
1,1 g (= 49 % d.Th.) der Titelverbindung werden chromatographisch und NMR-spektroskopisch rein erhalten;
Fp. 178°C.
Rf = 0,62 (Chloroform, Methanol, Eisessig = 90:10:5, Silicagel).

Die Herstellung des R,S S S-analogen Dimeren erfolgt nach der gleichen Vorschrift.
Fp. 174°C
Rf = 0,57 (Chloroform, Methanol, Eisessig = 90:10:5, Silicagel).

Die Ausgangsverbindungen werden wie folgt hergestellt:

N-(3-Phenyl-2-S-acetylmercaptopropanoyl)-S-alanin-methylester

Zu einer Lösung von 1,98 g (26 mMol) Thiolessigsäure in 70 ml wasserfreiem Ether werden unter Rühren, Kühlung auf 0°C und $N_2$-Atmosphäre 2,6 g (26 mMol) Triethylamin, gelöst in 20 ml wasserfreiem Ether, langsam zugetropft. Anschließend werden dieser Lösung 4,1 g (13 mMol) N-(3-Phenyl-2-R-brompropanoyl)-S-alanin-methylester (Herstellung s. Beispiel 1) in 50 ml wasserfreiem Ether tropfenweise zugesetzt und 90 Minuten am Rückfluß gekocht. Nach Abkühlen auf Raumtemperatur wird filtriert und das Lösungsmittel abdestilliert. Der ölige Rückstand (6,2 g) wird über Kieselgel mit n-Hexan, Essigester (2:1) chromatographiert und 3,9 g (96,9 % d.Th.) eines NMR-spektroskopisch einheitlichen, kristallinen Produkts als Titelverbindung erhalten.

N,N'-[2,2'-S-dithiobis-(3-phenylpropanoyl)]-bis-S-alanin

3,9 g (12,6 mMol) N-(3-Phenyl-2-S-acetylmercaptopropanoyl)-S-alanin-methylester werden in 40 ml Methanol gelöst und unter Rühren und Eiskühlung mit 37,8 ml (37,8 mMol) 1 n NaOH versetzt. Die Lösung wird 3 Stunden bei Raumtemperatur gerührt, dann mit einer 2%igen methanolischen Jodlösung bis zur bleibenden Gelbfärbung versetzt. Anschließend wird mit verdünnter $Na_2S_2O_3$-Lösung entfärbt, vom Lösungsmittel im Vakuum abdestilliert, der Rückstand in Wasser gelöst und mit Dichlormethan gewaschen. Die wäßrige Phase wird im Vakuum zur Entfernung des Dichlormethans eingeengt und mit konzentrierter HCl angesäuert. Das ausgefallene Reaktionsprodukt wird mit

Dichlormethan extrahiert, mit gesättigter NaCl-Lösung gewaschen, über $MgSO_4$ getrocknet und vom Lösungsmittel abdestilliert.
2,5 g (= 78,6 % d.Th.) der Titelverbindung können als erstarrter Schaum isoliert werden.
Rf = 0,5 (Chloroform, Methanol, Eisessig = 90:10:5, Silicagel).

Die Herstellung der R S S- und R,S S S-analogen Diastereomeren erfolgt nach dem gleichen Verfahren.

Beispiel 8

N-[N-(3-Phenyl-2-S-mercaptopropanoyl)-S-alanyl]-S-prolin-pivaloyloxymethylester

1,75 g (5 mMol) N-[N-(3-Phenyl-2-S-mercaptopropanoyl)-S-alanyl]-S-prolin werden in 30 ml Aceton gelöst und unter Rühren und Stickstoffatmosphäre nacheinander 500 mg (5 mMol) $KHCO_3$, 140 mg (0,84 mMol) KJ und 0,81 ml (5 mMol) Chlormethylpivalat zugesetzt. Die Mischung wird 3 Stunden am Rückfluß gekocht und nach Abkühlen abgesaugt. Das Filtrat wird im Vakuum eingeengt, der Rückstand in Essigester aufgenommen und nacheinander gewaschen mit Wasser, gesättigter $NaHCO_3$-Lösung, Wasser und gesättigter NaCl-Lösung. Man trocknet über $MgSO_4$ und destilliert das Lösungsmittel im Vakuum ab. Der ölige Rückstand (1,5 g = 69 % d.Th.) wird über Kieselgel chromatographiert (Laufmittel: Essigester, n-Hexan (2:1)) und dabei 2 Fraktionen erhalten:
0,7 g (= 32 % d.Th.) entsprechen der Titelverbindung;
Rf = 0,28.
0,3 g (13,7 % d. Th.) entsprechen dem S-Alkylierungsprodukt N-[N-(3-Phenyl-2-S-pivaloyloxymethylmercaptopropanoyl)-S-alanyl]-S-prolin pivaloyloxymethylester.

Die Herstellung der R S S- und R,S S S-analogen Diastereomeren sowie der entsprechenden S-Acyl-Verbindungen erfolgt nach der im Beispiel 11 beschriebenen Weise.

Beispiel 9

N-[N-(3-Phenyl-2-S-{4-chlor-3-sulfon-amidobenzoyl}-mercaptopropanoyl-S-alanyl]-S-prolin

Zu einer Lösung von 2,1 g (6 mMol) N-[N-(3-Phenyl-2-S-mercaptopropanoyl)-S-alanyl]-S-prolin in 100 ml Wasser und 1,008 g (12 mMol) $NaHCO_3$ werden 1,524 g (6 mMol) 4-Chlor-3-sulfamoylbenzoesäurechlorid gegeben und 2 Stunden bei Raumtemperatur weitergerührt. Nach 2-tägigem Stehen wird mit Dichlormethan gewaschen, die wäßrige Phase mit verdünnter HCl angesäuert und die ausgefallenen Kristalle abgesaugt.
1,4 g (= 82,1 % d.Th.) der Titelverbindung können als Festsubstanz isoliert werden.
Rf = 0,3 (Butanon, Aceton, Wasser = 60:6:10, Silicagel).
Die NMR-spektroskopischen Daten bestätigen die Struktur und die Reinheit der Substanz.

Beispiel 10

N-[N-(3-Phenyl-2-S-mercaptopropanoyl)-S-alanyl]-S-prolyl-S-phenylalanin

Zu einer Lösung von 815 mg (10,7 mMol) Thiolessigsäure in 35 ml wasserfreiem Ether werden unter $N_2$-Atmosphäre, Eiskühlung und Rühren langsam 1,08 g (10,7 mMol) Triethylamin zugetropft. Anschließend wird eine Lösung von 3 g (5,37 mMol) N-[N-(3-Phenyl-2-R-brompropanoyl)-S-alanyl]-S-prolyl-S-phenylalaninmethylester in 20 ml wasserfreiem Dichlormethan tropfenweise zur obigen

Lösung gegeben und 90 Minuten am Rückfluß gekocht. Nach dem Abkühlen wird abgesaugt und der Filterrückstand in Dichlormethan gelöst. Man wäscht mit gesättigter $NaHCO_3$-Lösung, verdünnter $KHSO_4$-Lösung, Wasser und gesättigter NaCl-Lösung. Nach dem Trocknen über $MgSO_4$ wird das Lösungsmittel abdestilliert und 3,3 g eines festen Rückstandes erhalten, der nach Umkristallisation aus Essigester eine Ausbeute von 2,03 g (= 68 % d.Th.) ergab.
Der Methylester hat einen Fp. von 157 - 158°C und einen Rf-Wert von 0,13 (Essigester, n-Hexan = 2:1, Silicagel).

Zur Verseifung werden zu einer Suspension von 2 g (3,6 mMol) des obigen Mercaptoacyl-methylesters in 50 ml Methanol und 20 ml Wasser unter Rühren und einer $N_2$-Atmosphäre 6 ml 5 n NaOH gegeben und 1,5 Stunden bei Raumtemperatur gerührt. Man gibt weitere 2 ml 5 n NaOH zu und rührt nochmals 1,5 Stunden weiter. Das Methanol wird im Vakuum abrotiert, der Rückstand mit Wasser verdünnt und zweimal mit Dichlormethan gewaschen.
Die wäßrige Phase wird mit verdünnter HCl-Lösung angesäuert und dreimal mit Dichlormethan extrahiert. Die organische Phase wird dann mit gesättigter NaCl-Lösung gewaschen, über $MgSO_4$ getrocknet und im Vakuum abdestilliert.
1,7 g (= 94,9 % d.Th.) der Titelverbindung werden als erstarrter Schaum erhalten, deren Struktur NMR-spektroskopisch bestätigt wurde.
Rf = 0,48 (Chloroform, Methanol, Eisessig = 90:10:5, Silicagel).

Nach dem gleich Verfahren werden die R,S S S - und R S S - analogen Diastereomeren erhalten.

Die Ausgangsverbindung wird wie folgt hergestellt:

N-[N-(3-Phenyl-2-R-brompropanoyl)-S-alanyl]-
S-prolyl-S-phenylalanin

Zu einer Lösung von 2,8 g (7 mMol) N-[N-(3-Phenyl-2-R-brompropanoyl)-S-alanyl]-S-prolin, 1,5 g (7 mMol) S-Phenylalaninmethylester-hydrochlorid und 708 mg (7 mMol) Triethylamin in 50 ml wasserfreiem Dichlormethan werden unter Rühren bei $0^{o}C$ 1,44 g (7 mMol) N,N'-Dicyclohexylcarbodiimid gegeben, 15 Minuten bei $0^{o}C$, dann über Nacht bei Raumtemperatur weitergerührt. Man filtriert, destilliert das Lösungsmittel im Vakuum ab und löst den Rückstand in Essigester. Dann wird die Lösung gekühlt, erneut filtriert und nacheinander gewaschen mit verdünnter $KHSO_4$-Lösung, gesättigter $NaHCO_3$-Lösung, Wasser, gesättigter NaCl-Lösung.
Es wird über $MgSO_4$ getrocknet und das Lösungsmittel im Vakuum abdestilliert. Der Rückstand (4 g) wird aus wenig Essigester umkristallisiert;
Ausbeute: 3 g (76,7 % d.Th.)
Rf = 0,37 (Essigester, n-Hexan = 2:1, Silicagel).

Beispiel 11

N-[N-(3-Phenyl-2-S-mercaptopropanoyl)-S-alanyl]-
S-prolyl-S-valin

Analog Beispiel 10 erhält man beim Einsatz von N-[N-(3-Phenyl-2-R-brompropanoyl)-S-alanyl]-S-prolyl-S-valin die Titelverbindung.
Rf = 0,48 (Chloroform, Methanol, Eisessig = 90:10:5, Silicagel).

Beispiel 12

N-[N-(3-Phenyl-2-S-mercaptopropanoyl)-S-alanyl]-S-prolyl-S-prolin

Analog Beispiel 10 erhält man beim Einsatz von N-[N-(3-Phenyl-2-R-brompropanoyl)-S-alanyl]-S-prolyl-S-prolin die Titelverbindung.
Rf = 0,26 (Essigsäureethylester, Silicagel).

Beispiel 13

N-[N-(3-Phenyl-2-S-mercaptopropanoyl)-S-alanyl]-S-thiazolidin-4-carbonsäure

Analog Beispiel 1 erhält man beim Einsatz von S-Thiazolidin-4-carbonsäure die Titelverbindung in Ausbeuten von 85,4 % d.Th..
Fp. 144 - 146°C
Rf = 0,46 (Chloroform, Methanol, Eisessig = 90:10:5, Silicagel).

Elementaranalyse: $C_{16}H_{20}N_2O_4S_2$

| | C | H | N | S |
|---|---|---|---|---|
| Berechnet: | 52,15 | 5,47 | 7,60 | 17,40 |
| Gefunden: | 51,96 | 5,41 | 7,46 | 17,41 |

Die Struktur ist NMR-spektroskopisch bestätigt.

Beispiel 14

N.[N-(3-Phenyl-2-S-mercaptopropanoyl)-S-alanyl]-4,5,6,7-tetrahydro-thieno[2,3-c]pyridin-7-carbonsäure

Analog Beispiel 1 erhält man beim Einsatz von 4,5,6,7-Tetrahydro-thieno[2,3-c]pyridin-7-carbonsäure in der R,S-, R- und S-Konfiguration die entsprechenden Diastereomeren der Titelverbindung.
Rf-Wert für das S S R,S-Diastereomerengemisch: 0,18 (Chloroform, Methanol, Eisessig = 90:10:5, Silicagel).

Beispiel 15

N-[N-(3-Phenyl-2-S-mercaptopropanoyl)-S-alanyl]-4,5,6,7-tetrahydro-thieno[3,2-c]pyridin-4-carbonsäure

Analog Beispiel 1 erhält man beim Einsatz von 4,5,6,7-Tetrahydro-thieno[3,2-c]pyridin-4-carbonsäuren die entsprechenden Diastereomeren der Titelverbindung.
Rf-Wert für das S S R,S-Diastereomerengemisch: 0,53 (Chloroform, Methanol, Eisessig = 90:10:5, Silicagel).

Beispiel 16

N-[N-(3-Phenyl-2-S-benzoylmercaptopropanoyl)-S-alanyl]-S-prolin

Zu einer Lösung von 2,1 g (15,4 mMol) Thiolbenzoesäure in 50 ml wasserfreiem Ether werden bei 0°C, Stickstoffatmosphäre und Rühren langsam 1,56 g (15,4 mMol) Triethylamin zugesetzt. Anschließend tropft man eine

Lösung von 3,5 g (7,7 mMol) N-[N-(3-Phenyl-2-R-brom-propanoyl)-S-alanyl]-S-prolin-tert.butylester in 30 ml wasserfreiem Ether zu und kocht 90 Minuten unter Rückfluß. Nach Absaugen wird die etherische Lösung mit verdünnter $KHSO_4$-Lösung, gesättigter $NHCO_3$-Lösung, Wasser und gesättigter NaCl-Lösung gewaschen, über $MgSO_4$ getrocknet und zur Trockne eingedampft. Der Rückstand (3,7 g) wird über Kieselgel chromatographiert (Elutionsmittel: Essigester, n-Hexan = 1:1) und 3,4 g (= 86,5 % d.Th.) des tert.Butylesters der Titelverbindung als erstarrter Schaum erhalten.
Rf = 0,27 (Essigester, n-Hexan = 1:1, Silicagel).

Zur Verseifung werden 3,4 g (6,6 mMol) des obigen tert.Butylesters in 34 ml Trifluoressigsäure und 17 ml Anisol gelöst und 2 Stunden bei Raumtemperatur gerührt. Im Vakuum wird die Reaktionslösung eingedampft und je dreimal mit Aceton und Chloroform gelöst und erneut im Vakuum einrotiert. Der Rückstand wird in Dichlormethan gelöst und mit Wasser und gesättigter NaCl-Lösung gewaschen. Nach Trocknen über $MgSO_4$ wird eingedampft und der Rückstand (3,1 g) über Kieselgel chromatographiert (Elutionsmittel: Dichlormethan, Methanol, Eisessig = 120:5:2).
2,2 g (= 73,3 % d.Th.) der Titelverbindung werden als farbloser, erstarrter Schaum erhalten.
Rf = 0,25 (Dichlormethan, Methanol, Eisessig = 120:5:2, Silicagel).

Beispiel 17

N-[N-(3-Phenyl-2-S-pivaloylmercaptopropanoyl)-S-alanyl-S-prolin

Gearbeitet wird wie im vorangehenden Beispiel beschrieben. Anstelle der Thiolbenzoesäure steht Thiolpivalinsäure. Die Umsetzung von 3,17 g (7 mMol) N-[N-(3-Phenyl-2-R-brompropanoyl)-S-alanyl]-S-prolin-tert.butylester und 1,65 g (14 mMol) Thiolpivalinsäure ergaben 3,3 g (= 96 % d.Th.) des tert.Butylesters der Titelverbindung. Rf = 0,34 (Essigester, n-Hexan = 1:1, Silicagel). Seine Verseifung mit Trifluoressigsäure führte zur Titelverbindung in einer Ausbeute von 2,4 g (= 82,4 % d.Th.).
Rf = 0,3 (Dichlormethan, Methanol, Eisessig = 120:6:2, Silicagel).

Beispiel 18

N-[N-(3-Phenyl-2-S-acetylmercaptopropanoyl)-S-alanyl]-S-prolin-pivaloyloxymethylester

2,3 g (5,8 mMol) N-[N-(3-Phenyl-2-S-acetylmercapto-propanoyl)-S-alanyl]-S-prolin (Zwischenprodukt aus Beispiel 1) werden in 40 ml wasserfreiem Aceton gelöst und unter Rühren und Stickstoffatmosphäre nacheinander 580 mg (5,8 mMol) $KHCO_3$, 161 mg (0,97 mMol) Kaliumjodid und 873 mg (5,8 mMol) Chlormethylpivalat zugesetzt. Die Mischung wird 3 Stunden am Rückfluß gekocht und nach Abkühlen abgesaugt. Das Filtrat wird im Vakuum eingeengt, der Rückstand in Essigester aufgenommen und der Reihe nach gewaschen mit Wasser, gesättigter $NaHCO_3$-Lösung, Wasser und gesättigter NaCl-Lösung.

Man trocknet über $MgSO_4$ und destilliert das Lösungsmittel im Vakuum ab. Der ölige Rückstand (2,7 g) wird über Kieselgel chromatographiert (Laufmittel: Essigester, n-Hexan = 2:1) und dabei 2,2 g (= 75 % d.Th.) der Titelverbindung als Öl erhalten.
Rf = 0,42 (Essigester, n-Hexan = 2:1, Silicagel).

Beispiel 19

N-[N-(3-Phenyl-2-S-benzoylmercaptopropanoyl)-S-alanyl]-S-prolin-pivaloyloxymethylester

Analog Beispiel 18 erhält man beim Einsatz von N-[N-(3-Phenyl-2-S-benzoylmercaptopropanoyl)-S-alanyl-S-prolin die Titelverbindung.
Rf = 0,43 (Essigester, n-Hexan = 1:1, Silicagel).

Beispiel 20

N-[N-(3-Phenyl-2-S-pivaloylmercaptopropanoyl)-S-alanyl]-S-prolin-pivaloyloxymethylester

Analog Beispiel 18 erhält man beim Einsatz von N-[N-(3-Phenyl-2-S-pivaloylmercaptopropanoyl)-S-alanyl]-S-prolin die Titelverbindung.
Rf = 0,45 (Essigester, n-Hexan = 2:1, Silicagel).

Beispiel 21

N-[N-(3-Phenyl-2-S-acetylmercaptopropanoyl)-S-alanyl]-N-cyclopentylglycin

6 g (20 mMol) N-(3-Phenyl-2-R-brompropanoyl)-S-alanin und 4 g (20 mMol) N-Cyclopentylglycin-tert.butylester werden in 100 ml Dichlormethan gelöst und unter Rühren und Kühlen (0°C) mit 4,1 g (20 mMol) N,N'-Dicyclohexylcarbodiimid versetzt und 15 Minuten bei dieser Temperatur, dann über Nacht bei Raumtemperatur weitergerührt. Nach Absaugen des Dicyclohexylharnstoffs wird das Lösungsmittel im Vakuum abdestilliert, der ölige Rückstand in Essigester gelöst und einige Zeit in der Kälte stehen gelassen. Nach erneutem Absaugen wird das Filtrat mit verdünnter $KHSO_4$-Lösung, gesättigter $NaHCO_3$-Lösung, Wasser und gesättigter NaCl-Lösung gewaschen und nach Trocknen über $MgSO_4$ im Vakuum abdestilliert. Der ölige Rückstand (6,7 g) wird über Kieselgel chromatographiert (Laufmittel: Essigester, n-Hexan = 1:2).
Ausbeute 4,3 g (= 44,6 % d.Th.) Brom-tert.Butylester als farbloses Öl werden erhalten.
Rf = 0,28 (Essigester, n-Hexan = 1:2, Silicagel).

Der Austausch des Broms gegen Thiolessigsäure und die anschließende Verseifung des tert.Butylesters zur Titelverbindung erfolgt in der im Beispiel 1 beschriebenen Weise.
$R_f$= 0,55 (Chloroform, Methanol, Eisessig = 90:10:5)

## Beispiel 22

### N-[N-(3-Phenyl-2-S-methylaminothiocarbonyl-mercaptopropanoyl)-S-alanyl]-S-prolin

Eine Lösung von 1,73 g (4,96 mMol) N-[N-(3-Phenyl-2-S-mercaptopropanoyl)-S-alanyl]-S-prolin in 10 ml 0,5 n NaOH und 25 ml Pyridin wird unter Stickstoffatmosphäre mit 0,4 g (5,47 mMol) Methylisothiocyanat versetzt und 2 Stunden auf 40°C erwärmt. Danach wird im Vakuum zur Trockne eingedampft, der Rückstand mit Wasser aufgeschlämmt und mit konzentrierter HCl angesäuert. Die wäßrige Phase wird mit Essigester

extrahiert, mit NaCl-Lösung gewaschen und über $MgSO_4$ getrocknet. Der Essigesterextrakt wird zur Trockne eingedampft und dabei 1,5 g (71,4 % d.Th.) der Titelverbindung als erstarrter Schaum erhalten, der über Kieselgel chromatographisch gereinigt wird.
(Laufmittel: Toluol, Essigsäure = 75:25).
Rf = 0,47 (Chloroform, Methanol, Eisessig = 90:10:5, Silicagel).

Beispiel 23

N-[N-(3-Phenyl-2-S-ethylaminocarbonyl-mercaptopropanoyl)-S-alanyl]-S-prolin

Eine Lösung von 1,72 g (4,92 mMol) N-[N-(3-Phenyl-2-S-mercaptopropanoyl)-S-alanyl]-S-prolin in 5 ml 1 n NaOH und 5 ml Pyridin wird mit 0,45 ml (5,7 mMol) Ethylisocyanat versetzt und 4 Stunden bei 40°C unter $N_2$-Atmosphäre gerührt. Danach wird im Vakuum zur Trockne eingedampft. Der Rückstand wird in Wasser aufgeschlämmt, mit 0,1 n HCl angesäuert und, wie im voranstehenden Beispiel beschrieben, weiterverarbeitet.
1,7 g (82,1 % d.Th.) der Titelverbindung werden als erstarrter Schaum erhalten.
Rf = 0,45 (Chloroform, Methanol, Eisessig = 90:10:5, Silicagel).

NMR-Daten für mehrere erfindungsgemäße Verbindungen wie folgt:

Beispiel 1

$^{1}H$ NMR daten ($CD_3OD$)

$\delta$ = 1,31, d, J = 7Hz , 3H, $\underline{CH_3}$-CH-

1,80 - 2,41, m, 4H, Pro - $CH_2CH_2$

3,16, m, 2H, Ø - $\underline{CH_2}$-CH-

3,43 - 3,89, m, 3H, Pro-$NCH_2$, Ø-$CH_2$-$\underline{CH}$-

4,41, m, 1H, Pro $\alpha$ CH

4,61, m, 1H, $CH_3$-$\underline{CH}$-

7,24, m, 5H, Aryl-H

S$\underline{H}$, N$\underline{H}$, COO$\underline{H}$, im Lösungsmittel-Blindpeak.

Beispiel 2

$^{1}H$ NMR Daten ($CDCl_3$)

δ = 1,15, d, J = 7Hz, 3H, $CH_3$-CH-

1,77 - 2,39, m, 4H, Pro $CH_2CH_2$-

2,05, d, J = 10Hz, SH

3,13, m, 2H, Ø-$CH_2$-CH-

3,43, m, 1H, Ø-$CH_2$-CH-

3,63, m, 2H, Pro-N-$CH_2$-

4,55, m, 1H, Pro α H

4,77, m, 1H, $CH_3$-CH-

7,25, m, 5H, Aryl-H

8,93, s, breit, COOH

Beispiel 4

$^{1}H$ NMR daten ($CD_3OD$)

δ = 1,35, d, 3H, J = 7Hz, $CH_3$-CH-

1,81 - 2,52, m, 4H, Pro-$CH_2CH_2$-

3,17, s, 2H, $SCH_2$-C(=O)

3,69, m, 2H, Pro N-$CH_2$

4,47, m, 1H, Pro α-CH-4,64, qu, 1H, J = 7Hz, $CH_3$-CH-

SH, NH and COOH im Lösungsmittel-Blindpeak

Beispiel 5

$^1$H-NMR ($CD_3OD$)

$\delta$ = 1,32, d, J = 7Hz, 3H, $CH_3$-CH-
1,77 - 2,45, m, 4H, Pro-$CH_2CH_2$-
3,65, m, 2H, Pro-N-$CH_2$
4,48, m, 1H, Pro α CH-
4,66, m, 1H, $CH_3$-CH-
4,82, s, 1H, Ø-CH-
7,24 - 7,59, m, 5H, Aryl-H
SH, NH, COOH im Lösungsmittel-Blindpeak

Beispiel 6

$\delta$ = 1,35, d, 3H, $CH_3$-CH-, J = 7Hz
2,08, m, 2H, Ø-$CH_2$-$CH_2$-
1,80 - 2,44, m, 4H, Pro-$CH_2$-$CH_2$-
2,72, m, 2H, Ø-$CH_2$-
3,26, m, 1H, Ø-$CH_2CH_2$-CH-
3,65, m, 2H, Pro-N$CH_2$-
4,50, m, 1H, Pro α CH-
4,77, m, 1H, $CH_3$-CH-
7,23, m, 5H, Aryl-H
7,50, m, 1H, NH,
8,96, s, 2H, SH COOH

Beispiel 7

$^1$H-NMR Daten ($CDCl_3$)

δ = 1,28, d, J = 7Hz, 6H, $CH_3$-CH-
1,66 - 2,51, m, 8H, Pro-$CH_2CH_2$-
3,06, m, 4H, Ø-$CH_2$-CH-
3,33 - 4,01, m, 6H, Pro-$NCH_2$, Ø-$CH_2$-CH-
4,42, m, 2H, Pro α CH
4,75, m, 2H, $CH_3$-CH-
6,75, s, 2H, COOH
7,21, m, 10H, Aryl-H
7,50, d, J = 8Hz, 2H, NH

Beispiel 8 - Titelverbindung

$^1$H-NMR Daten ($CDCl_3$)

δ = 1,20, s, 9H, $C(CH_3)_3$
1,34, d, J = 7Hz, 3H, $CH^3$-CH-
1,86, d, J = 10Hz, 1H, SH
1,82 - 2,42, m, 4H, Pro-$CH_2CH_2$-
2,91 - 3,81, m, 5H, Pro N-$CH_2$, Ø-$CH_2$CH-
4,49, m, 1H, Pro α CH
4,66, m, 1H, $CH_3$-CH-
5,75, m, 2H, O-$CH_2$-O
7,23, m, 6H, 5 Aryl-H, NH

Beispiel 8

N-/N-3-Phenyl-2-S-pivaloyloxymethylmercaptopropanoyl)-S-alanyl/-S-thiazolidin-4-carbonsäure

$^1$H-NMR Daten ($CDCl_3$)

$\delta$ = 1,16, s, 9H, $C(CH_3)_3$
1,21, s, 9H, $C(CH_3)_3$
1,33, d, J = 7Hz, 3H, $CH_3$-CH-
1,78 - 2,43, m, 4H, Pro-$CH_2CH_2$
3,14, m, 2H, Ø-$CH_2$-CH-
3,34 - 3,89, m, 3H, Ø-$CH_2$-CH-, Pro-$NCH_2$
4,36 - 4,89, m, 2H, Pro α CH, $CH_3$-CH-
5,75, m, 2H, O-$CH_2$-O
7,14, d, J = 8Hz, 1H, NH
7,23, m, 5H, Aryl-H

Beispiel 12

$^1$H-NMR Daten ($CDCl_3$)

$\delta$ = 1,34, d, J = 7Hz, 3H, $CH_3$-CH-
1,97, d, J = 10Hz, 1H, SH
1,71 - 2,43, m, 8H, Pro $CH_2CH_2$
2,84 - 4,00, m, 7H, Pro $NCH_2$, Ø-$CH_2$-CH-
4,26 - 4,96, m, 3H, Pro α CH, $CH_3$-CH-
6,20, s, 2H, NH, COOH
7,26, m, 5H, Aryl-H

## Beispiel 13

$^1$H-NMR Daten ($CDCl_3$)

$\delta$ = 1,35, d, J = 7Hz, 3H, $CH_3$-CH-
2,03, d, J = 10Hz, 1H, SH
3,20, m, 2H, Ø-$CH_2$-CH-
3,27, d, 2H, S-$CH_2$-CH-, J = 7Hz
3,50, m, 1H, Ø-$CH_2$-CH-
4,72, m, 2H, N-$CH_2$-S
4,77, m, 1H, $CH_3$-CH-
5,10, t, J = 7Hz, $SCH_2$-CH-
6,52, s, 1H, COOH
7,27, m, 5H, Aryl-N
7,78, d, J = 8Hz, 1H, NH

## Beispiel 14

$^1$H-NMR Daten ($CD_3OD$)

$\delta$ = 1,33, d, J = 7Hz, 3H, $CH_3$-CH-
2,50 - 3,91, m, 7H, Ø-$CH_2$-CH-, -$CH_2$-$CH_2$-
4,99, m, 1H, $CH_3$-CH-
5,85, s, 1H, N-CH-C=O
6,84, d, 1H, thiophen 4H
7,34, d, 1H, " 5H
7,18, m, 5H, Aryl-H
SH, NH, COOH im Lösungsmittel-Blindpeak

Beispiel 15

$^1$H-NMR Daten ($CDCl_3$)

δ = 1,34, d, J = 7Hz, 3H, $\underline{CH_3}$-CH-
2,02, d, J = 9Hz, 1H, $\underline{SH}$
2,72 - 4,43, m, 7H, ø-$\underline{CH_2}$-$\underline{CH}$-, -$CH_2$-$CH_2$-
5,00, m, 1H, $CH_3$-$\underline{CH}$-
5,91, s, 1H, $\underline{CH}$-COOH
7,23, m, 9H, Aryl-H, thiophen—H, N$\underline{H}$, COOH

Beispiel 16

$^1$H-NMR Daten ($CDCl_3$)

δ = 1,28, d, 3H, J = 7,0Hz, $\underline{CH_3}$-CH-
1,77 - 2,39, m, 4H, Pro-$CH_2CH_2$-
3,24, m, 2H, ø-$\underline{CH_2}$-CH-
3,58, m, 2H, Pro-$NCH_2$
4,47, m, 1H, ø-$CH_2$-$\underline{CH}$-
4,53, m, 1H, Pro α-$\underline{CH}$-
4,75, m, 1H, $CH_3$-$\underline{CH}$-
7,02 - 8,00, m, 10H, 2-aromatics
8,50, s, 1H, COO$\underline{H}$

Beispiel 17

$^{1}H$-NMR Daten ($CDCl_3$)

$\delta$ = 1,17, s, 9H, $C(CH_3)_3$

1,26, d, 3H, J = 7Hz, $\underline{CH_3}$-CH-

1,82 - 2,27, m, 4H, Pro-$CH_2CH_2$-

3,14, m, 2H, Ø-$\underline{CH_2}$-CH-

3,57, m, 2H, Pro N-$CH_2$-

4,19, m, 1H, Ø-$CH_2$-$\underline{CH}$-

4,50, m, 1H, Pro α-$\underline{CH}$-

4,73, m, 1H, $CH_3$-$\underline{CH}$-

6,98, d, 1H, J = 8Hz, -NH

7,21, m, 5H, Aryl-H

8,30, s, 1H, COO$\underline{H}$

Beispiel 18

$^1$H-NMR Daten ($CDCl_3$)

$\delta$ = 1,21, s, 9H, $C(CH_3)_3$

1,23, d, 3H, J = 7Hz, $\underline{CH_3}$-CH-

1,77 - 2,27, m, 4H, Pro $CH_2CH_2$-

2,30, s, 3H, $CH_3$-C=O

3,15, m, 2H, ~~O~~-$\underline{CH_2}$-CH-

3,58, m, 2H, Pro-$NCH_2$

4,15, m, 1H, ~~O~~-$CH_2$-$\underline{CH}$-

4,48, m, 1H, Pro α $\underline{CH}$-

4,67, m, 1H, $CH_3$-$\underline{CH}$-

5,75, m, 2H, -O-$CH_2$-O-

6,92, d, 1H, J = 8Hz, $\underline{NH}$

7,23, m, 5H, Aryl-H.

Beispiel 19

$^{1}H$-NMR Daten ($CDCl_3$)

δ = 1,19, s, 9H, $C(CH_3)_3$
1,31, d, 3H, $CH_3$-CH-, J = 7 Hz
1,80 - 2,34, m, 4H, Pro-$CH_2CH_2$-
3,26, m, 2H, Ø-$CH_2$-CH-
3,58, m, 2H, Pro-N $CH_2$-
4,48, m, 1H, Ø-$CH_2$-CH-
4,50, m, 1H, Pro α CH-
4,73, m, 1H, $CH_3$-CH-
7,00, d, 1H, J = 7Hz, NH-
5,76, m, 2H, O-$CH_2$-O
7,27, m, 5H, Aryl-H
7,34 - 8,02, m, 5H, Ø-C(=O)-H

0174571

Beispiel 20

$^1H$-NMR Daten ($CDCl_3$)

$\delta$ = 1,16, s, 9H, $C(CH_3)_3$
1,20, s, 9H, $C(CH_3)_3$
1,27, d, 3H, J = 7Hz, $\underline{CH_3}$-CH-
1,82 - 2,32, m, 4H, Pro $CH_2CH_2$
3,16, m, 2H, Phe-$\underline{CH_2}$-CH-
3,57, m, 2H, Pro N $CH_2$-
4,16, m, 1H, Ø-$CH_2$-$\underline{CH}$-
4,48, m, 1H, Pro α $\underline{CH}$-
4,71, m, 1H, $CH_3$-$\underline{CH}$-
5,76, m, 2H, O-$CH_2$-O
6,82, d, 1H, J = 8 Hz, $\underline{NH}$
7,22, m, 5H, Aryl-H

Beispiel 21

$^{1}H$-NMR Daten ($CDCl_3$)

δ = 1,31, d, 3H, J = 7Hz, $\underline{CH_3}$-CH-

1,16 - 2,11, m, 8H, cyclopentyl-$\underline{CH_2}$

2,28, s, 3H, $CH_3$-C=O

3,15, m, 2H, ⌀-$\underline{CH_2}$-CH-

2,92, m, 2H, N-$CH_2$-C=O

4,22, m, 1H, cyclopentyl-$\underline{CH}$

4,26, m, 1H, ⌀-$CH_2$-$\underline{CH}$-

4,92, m, 1H, $CH_3$-$\underline{CH}$-

5,61, s, 1H, COO$\underline{H}$

7,18, d, 1H, J = 8Hz, NH

7,24, m, 5H, Aryl-H

Beispiel 22

$^1H$-NMR Daten ($CDCl_3$)

δ = 1,27, d, J = 7Hz, 3H, $CH_3$-CH-

1,73 - 2,41, m, 4H, Pro-$CH_2$-$CH_2$-

3,18, d, J = 4Hz, 3H, NH-$CH_3$

3,21, m, 2H, Ø-$CH_2$-CH-

3,57, m, 2H, Pro N-$CH_2$

4,48, m, 1H, Pro α-CH

4,65, m, 1H, $CH_3$-CH-

4,48, m, 1H, Ø-$CH_2$-CH-

7,24, m, 5H, Aryl-H

7,47, d, J = 7Hz, NH-CH-8,48, qu, J = 4Hz, NH-$CH_3$

8,77, s, 1H, COOH

Beispiel 23

$^1$H-NMR Daten ($CDCl_3$)

δ = 1,11, t, J = 7Hz, 3H, $\underline{CH_3}$-$CH_2$

1,28, d, J = 7Hz, 3H, $\underline{CH_3}$-CH

1,77 - 2,41, m, 4H, Pro $CH_2CH_2$

3,15, m, 2H, Ø-$\underline{CH_2}$-CH-

3,23, m, 2H, N-$\underline{CH_2}$-$CH_3$

3,56, m, 2H, Pro-N-$CH_2$

4,19, m, Ø-$CH_2$-$\underline{CH}$-

4,52, m, Pro α CH

4,73, m, $CH_3$-$\underline{CH}$-

5,65, s, breit, 2xNH and COO$\underline{H}$

7,23, m, 5H, Aryl-H

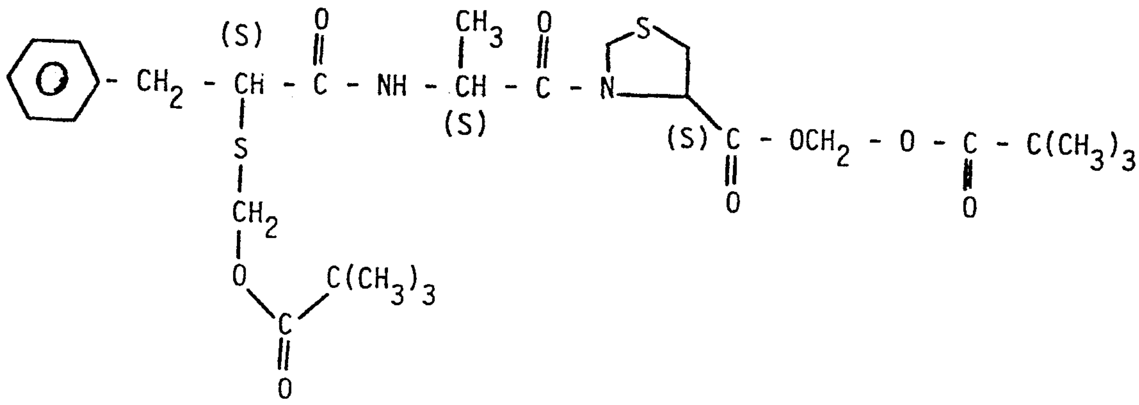

$^1$H-NMR Daten ($CDCl_3$)

$\delta$ = 1,15, s, 9H, $C(CH_3)_3$

1,22, s, 9H, $C-(CH_3)_3$

1,36, d, J = 7Hz, 3H, $\underline{CH_3}$-CH-

2,83 - 3,49, m, 4H, ~~O~~-$\underline{CH_2}$-CH-, S-$\underline{CH_2}$-CH-

3,74, m, 1H, ~~O~~-$CH_2$-$\underline{CH}$-

4,39 - 4,84, m, 3H, N-$CH_2$-S, $CH_3$-$\underline{CH}$-

5,07, m, 1H, S-$CH_2$-$\underline{CH}$-

5,76, m, 2H, O-$CH_2$-O

7,23, m, 6H, Aryl-H, $\underline{NH}$

Rf = 0,48 (Essigester, n-Hexan = 1:1)

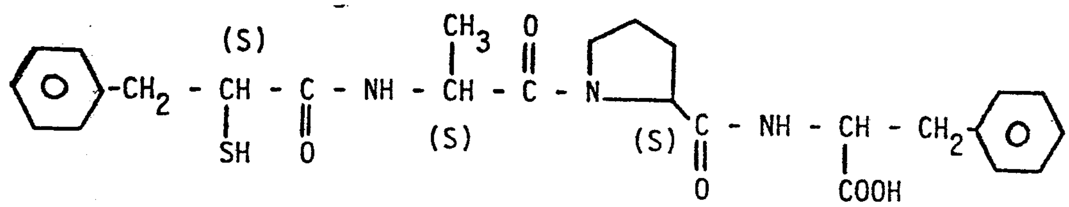

$^1$H-NMR Daten (CDCl$_3$)

$\delta$ = 1,05, d, J = 7Hz, 3H, $CH_3$-CH-

2,08, d, J = 10Hz, 1H, SH

1,59 - 2,25, m, 4H, Pro-$CH_2CH_2$

2,84 - 3,93, m, 7H, Pro-$NCH_2$, $\phi$-$CH_2$-CH-; $\phi$-$CH_2$-CH-COOH

4,46 - 4,99, m, 3H, Pro $\alpha$ CH, $CH_3$-CH-, $\phi$-$CH_2$-CH-COOH

7,24, m, 12H, Aryl-H, NH, COOH

7,75, d, J = 8Hz, NH

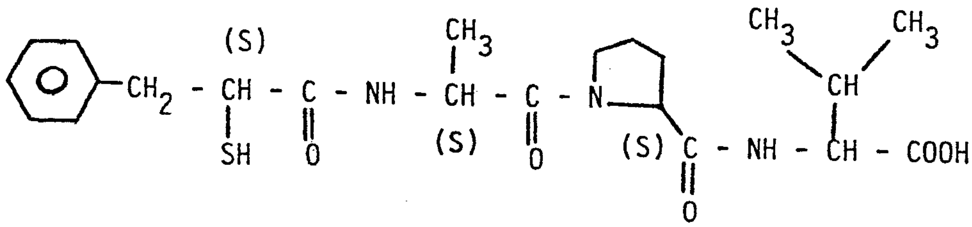

$^1$H-NMR Daten ($CDCl_3$)

$\delta$ = 0,86, d, J = 7Hz, 6H, Isobutyl-$CH_3$
1,32, d, J = 7Hz, 3H, $CH_3$-CH-
2,09, d, J = 10Hz, 1H, SH
1,71 - 2,70, m, 5H, Pro-$CH_2CH_2$, Isobutyl-CH-$(CH_3)_2$
3,14, m, 2H, Ø-$CH_2$-CH-
3,41 - 4,00, m, 3H, Pro-$NCH_2$, Ø-$CH_2$-CH-
4,34 - 4,99, m, 3H, Pro α CH, $CH_3$-CH-, Isobutyl-CH
6,58, s, 1H, COOH
7,22, m, 5H, Aryl-H
7,53, d, J = 10Hz, 1H, NH-CH-$CH_3$
7,75, d, J = 8Hz, 1H, NH-Isobutyl

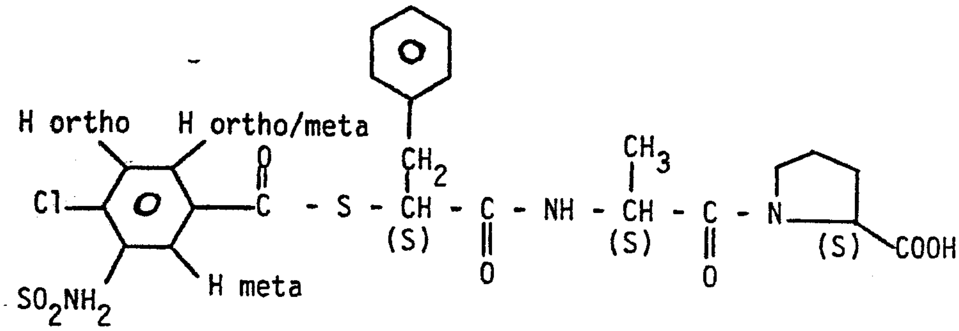

$^1$H-NMR Daten ($CD_3OD$)

$\delta$ = 1,30, d, J = 7Hz, 3H, $\underline{CH_3}$-CH-

1,78 - 2,40, m, 4H, Pro $CH_2CH_2$

2,90 - 3,42, m, 2H, $\phi$-$\underline{CH_2}$-CH-

3,56, m, 2H, Pro N-$CH_2$

4,27 - 4,84, m, 3H, Pro $\alpha$ CH, $\phi$-$CH_2$-$\underline{CH}$-, $CH_3$-$\underline{CH}$-

7,27, m, 5H, Aryl-H

7,73, d, J = 8Hz, 1H, ortho-Aryl-H

8,08, dd, J = 8Hz, 3Hz, 1H, ortho/meta Aryl-H

8,57, d, J = 3Hz, 1H, meta-Aryl-H

$\underline{NH_2}$, $\underline{NH}$, COO$\underline{H}$ im Lösungsmittel-Blindpeak

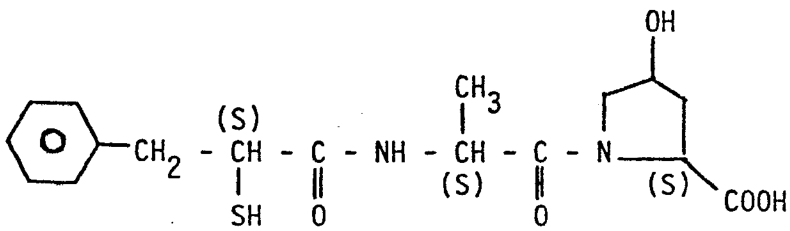

$^1$H-NMR Daten ($CDCl_3$)

$\delta$ = 1,33, d, J = 7Hz, 3H, $CH_3$-CH-
2,02, d, J = 10Hz, 1H, SH
2,00 - 2,50, m, 2H, Pro ß-$CH_2$-
2,84 - 3,66, m, 3H, Ø-$CH_2$-CH-
3,48 - 3,89, m, 2H, Pro-N-$CH_2$
4,50, m, 2H, Pro α-CH, CH-OH
5,40, s, breit, OH, COOH
7,24, m, 5H, Aryl-H
7,46, d, J = 8Hz, 1H, NH

Rf = 0,23 (Chloroform, Methanol, Eisessig = 90:10:5)

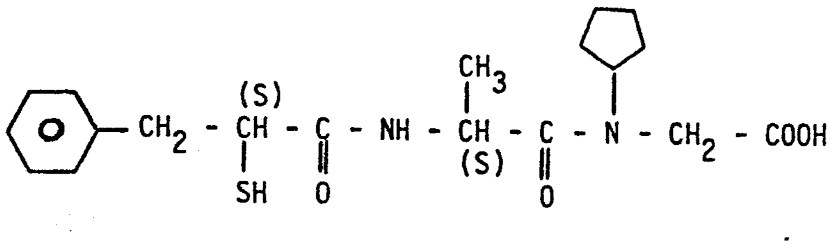

$^1$H-NMR Daten ($CDCl_3$)

δ = 1,34, d, 3H, J = 7Hz, $CH_3$-CH-

1,34 - 1,99, m, 8H, cyclopentyl-$CH_2$-

2,00, d, J = 10Hz, SH

3,16, m, 2H, Ø-$CH_2$-CH-

3,53, m, 1H, Ø-$CH_2$-CH-

3,92, m, 2H, N-$CH_2$-C=O

4,28, m, 1H, cyclopentyl-CH

4,99, m, 1H, $CH_3$-CH-

5,91, s, 1H, COOH

7,24, m, 5H, Aryl-H

7,46, d, 1H, J = 8Hz, NH

Rf = 0,63 (Chloroform, Methanol, Eisessig = 90:10:5)

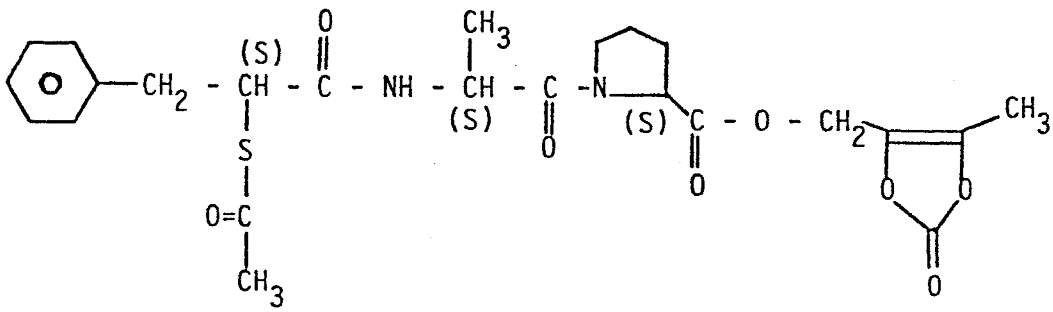

$^1$H-NMR Daten ($CDCl_3$)

$\delta$ = 1,29, d, (3H), J = 7Hz, $CH_3$-CH-
1,76 - 2,31, m, 4H, Pro-$CH_2$-$CH_2$
2,16, s, 3H, $CH_3$-C=
2,31, s, 3H, $CH_3$-$\underset{\underset{O}{\|}}{C}$-
3,13, m, 2H, Ø-$CH_2$
3,58, m, 2H, Pro N-$CH_2$-
4,24, m, 1H, Ø-$CH_2$-CH-
4,48, m, 1H, $CH_3$-CH-
4,58, m, 1H, Pro $\alpha$ CH-
4,78, m, 2H, O-$CH_2$-
6,87, d, 1H, J = 7,5Hz, NH
7,23, m, 5H, Aryl-H

Rf = 0,17 (Essigester, Hexan = 2:1)

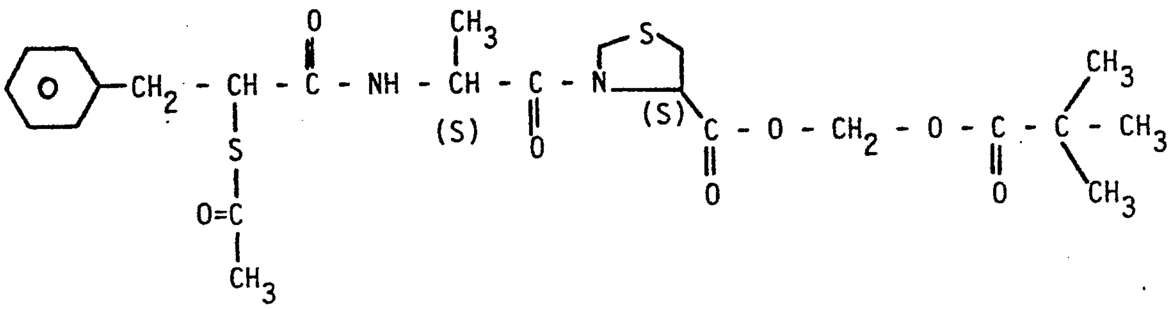

$^1$H-NMR Daten ($CDCl_3$)

δ = 1,19, s, 9H, $C(CH_3)_3$
1,32, d, J = 7Hz, 3H, $CH_3$-CH-
2,30, s, 3H, $CH_3$-C=O
3,11, m, 2H, Ø-$CH_2$-CH-
3,22, m, 2H, S-$CH_2$-CH-
4,12, m, 1H, Ø-$CH_2$-CH-
4,59, m, 2H, N-$CH_2$-S-
4,64, m, 1H, $CH_3$-CH-
5,07, m, 1H, N-CH-C=O
5,74, m, 2H, O-$CH_2$-O
6,86, d, J = 8Hz, 1H, NH
7,18, m, 5H, Aryl-H

Rf = 0,35 (Essigester, n-Hexane = 1:1)

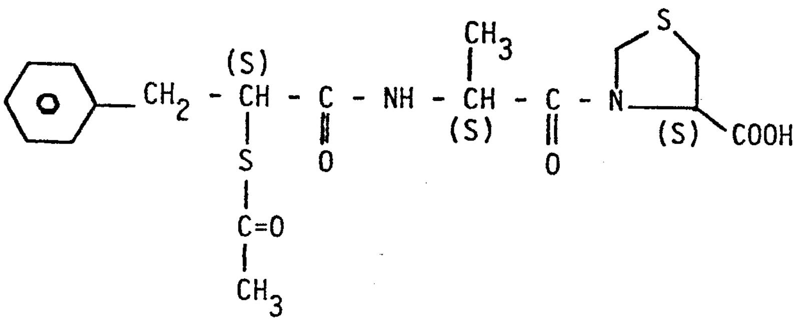

$^{1}$H-NMR Daten ($CDCl_3$)

$\delta$ = 1,32, d, 3H, J = 7Hz, $\underline{CH_3}$-CH-
2,30, s, 3H, $CH_3$-C=O
3,14, m, 2H, Ø-$\underline{CH_2}$-CH-
3,25, d, 2H, J = 6Hz, S$\underline{CH_2}$-CH-
4,25, m, 1H, Ø-$CH_2$-$\underline{CH}$-
4,65, m, 2H, N-$\underline{CH_2}$-S
4,78, m, 1H, $CH_3$-$\underline{CH}$-
5,07, t, 1H, J = 6 Hz, N-$\underline{CH}$-COO
7,16, d, 1H, J = 8Hz, NH
7,20, m, 5H, Aryl-H
8,48, s, 1H, COO$\underline{H}$

Rf= 0,47 (Chloroform, Methanol, Eisessig = 90:10:5)

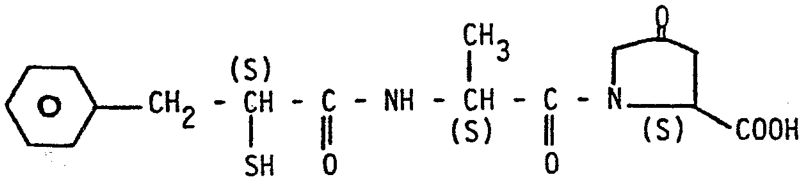

$^1$H-NMR Daten ($CDCl_3$)

$\delta$ = 1,30, d, J = 7Hz, 3H, $CH_3$-CH-

2,09, d, J = 10Hz, 1H, SH

2,48 - 3,81, m, 5H, ø-$CH_2$-CH-, $CH_2$-C=O

4,13, m, 2H, N-$CH_2$

4,64, m, 1H, $CH_3$-CH-

4,99, m, 1H, >CH-COOH

7,24, m, 5H, Aryl-H

7,57, d, J = 8Hz, 1H, NH

9,69, s, 1H, COOH

Rf = 0,34 (Chloroform, Methanol, Eisessig = 120:5:2)

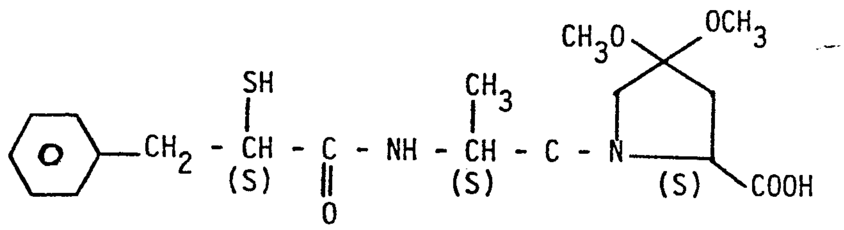

$^1$H-NMR Daten ($CDCl_3$)

δ = 1,31, d, 3H, J = 7Hz, $CH_3$-CH-
2,05, d, 1H, J = 10Hz, SH
2,35, d, 2H, J = 7Hz, Pro-$CH_2$-CH-
3,19, m, 2H, ~~O~~-$CH_2$-CH-
3,27, s, 6H, 2 $OCH_3$
3,50, m, 1H, ~~O~~-$CH_2$-CH-
3,68, m, 2H, Pro-N-$CH_2$
4,59, m, 1H, Pro α-CH-
4,70, m, 1H, $CH_3$-CH-
5,95, s, breit, 2H, NH und COOH
7,23, m, 5H, Aryl-H

Rf = 0,49 (Chloroform, Methanol, Eisessig = 90:10:5)

36z

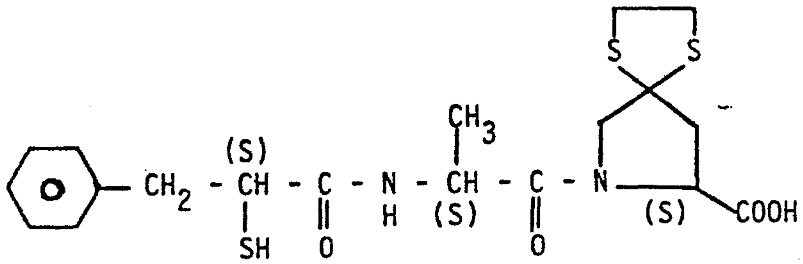

$^1$H-NMR Daten ($CDCl_3$)

$\delta$ = 1,30, d, J = 7Hz, 3H, $\underline{CH_3}$-CH-

2,02, d, J = 10Hz, 1H, S$\underline{H}$

2,67, m, 2H, Pro-$\underline{CH_2}$-CH-

3,15, m, 2H, Ø-$\underline{CH_2}$-CH-

3,36, s, 4H, -$SCH_2CH_2$-S-

3,55, m, 1H, Ø-$CH_2$-$\underline{CH}$-

4,00, m, 2H, Pro-N-$CH_2$

4,64, m, 2H, $CH_3$-$\underline{CH}$-, Pro $\alpha$-$\underline{CH}$-

7,23, m, 5H, Aryl-H

7,41, d, J = 8Hz, 1H, N$\underline{H}$

7,56, s, 1H, COO$\underline{H}$

Rf = 0,5 (Chloroform, Methanol, Eisessig 90:10:5)

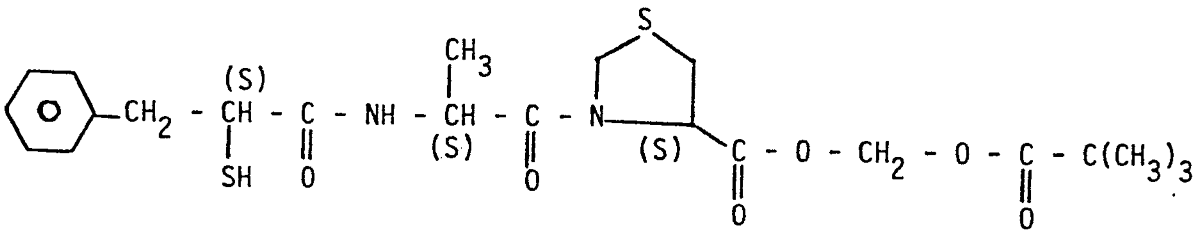

$^1$H-NMR Daten ($CDCl_3$)

$\delta$ = 1,18, s, 9H, $C(CH_3)_3$
1,34, d, J = 7Hz, 3H, $CH_3$-CH-
1,99, d, J = 10Hz, 1H, SH
2,93 - 3,77, m, 5H, ~~O~~-$CH_2$-CH-, $SCH_2$-CH-
4,41 - 4,94, m, 3H, N-$CH_2$-S, $CH_3$-CH-
5,07, m, 1H, S-$CH_2$-CH-
5,77, m, 2H, $OCH_2$-O
7,07, d, J = 9Hz, 1H, NH
7,23, m, 5H, Aryl-H

Rf = 0,33 (Eisessig, n-Hexan = 1:1)

## Pharmazeutische Anwendungsbeispiele

### a) Dragees

1 Drageekern enthält:

| | |
|---|---|
| Wirkstoff gemäß Anspruch 1 | 10,0 mg |
| Milchzucker | 60,0 mg |
| Maisstärke | 35,0 mg |
| Gelatine | 3,0 mg |
| Magnesiumstearat | 2,0 mg |
| | 110,0 mg |

Herstellung:

Die Mischung der Wirksubstanz mit Milchzucker und Maisstärke wird mit einer 10%igen wässrigen Gelatinelösung durch ein Sieb mit 1 mm Maschenweite granuliert, bei 40°C getrocknet und nochmals durch ein Sieb gerieben. Das so erhaltene Granulat wird mit Magnesiumstearat gemischt und verpreßt. Die so erhaltenen Kerne werden in üblicher Weise mit einer Hülle überzogen, die mit Hilfe einer wässrigen Suspension von Zucker, Titandioxyd, Talkum und Gummi arabicum aufgebracht wird. Die fertigen Dragees werden mit Bienenwachs poliert.

b) Tabletten

| | |
|---|---|
| Wirkstoff gemäß Anspruch 1 | 10,0 mg |
| Milchzucker | 70,0 mg |
| Maisstärke | 50,0 mg |
| lösliche Stärke | 7,0 mg |
| Magnesiumstearat | 3,0 mg |
| | 140,0 mg |

Herstellung:

Wirkstoff und Magnesiumstearat werden mit einer wässrigen Lösung der löslichen Stärke granuliert, das Granalat getrocknet und innig mit Milchzucker und Maisstärke vermischt. Das Gemisch wird sodann zu Tabletten von 230 mg Gewicht verpreßt, die je 100 mg Wirkstoff enthalten.

c) Injektionslösungen

| | | |
|---|---|---|
| Wirkstoff gemäß Anspruch 1 | | 5,0 mg |
| Äthanolamin | | 60,0 mg |
| Natriumchlorid | | 20,0 mg |
| destilliertes Wasser | ad | 2 ml |

Herstellung:

Der Wirkstoff und die Hilfsstoffe werden in einer ausreichenden Menge destilliertem Wasser gelöst und mit der erforderlichen Menge Wasser auf die gewünschte Konzentration gebracht. Die Lösung wird filtriert und unter aseptischen Bedingungen in 2 ml Ampullen abgefüllt. Die Ampullen werden sterilisiert und verschlossen. Jede Ampulle enthält 5 mg Wirkstoff.

d) Kapseln

| | |
|---|---|
| Wirkstoff gemäß Anspruch 1 | 10,0 mg |
| Milchzucker | 250,0 mg |
| Maisstärke | 40,0 mg |
| Talk | 10,0 mg |
| | 310,0 mg |

Wirkstoff, Milchzucker und Maisstärke werden zunächst in einem Mischer und dann in einer Zerkleinerungsmaschine vermengt. Das Gemisch wird nochmals in den Mischer gegeben, gründlich mit dem Talk vermengt und maschinell in Hartgelatinekapseln abgefüllt.

e) Suppositorien

| | |
|---|---|
| Wirkstoff gemäß Anspruch 1 | 0,1 g |
| Kakaobutter (Fp. 36-37°C) | 1,6 g |
| Carnaubawachs | 0,1 g |
| | 1,8 g |

Kakaobutter und Carnaubawachs werden geschmolzen, gründlich vermengt und auf 45°C abgekühlt. In diese Masse wird der feinpulverisierte Wirkstoff eingerührt. Anschließend wird die Mischung in leicht vorgekühlten Suppositorienformen geeigneter Größe gegossen und abkühlen gelassen.

## P a t e n t a n s p r ü c h e

1. Aminosäurederivate der allgemeinen Formel

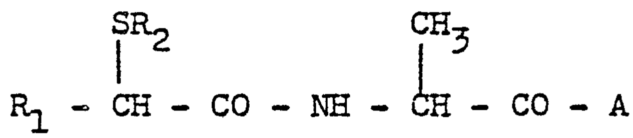

(I)

worin

$R_1$ H, Phenyl, Benzyl, Phenethyl;

$R_2$ H, Acetyl, Benzoyl, 3-Sulfonamido-4-chlor-benzoyl, 3-Sulfonamido-4-chlor-6-hydroxy-benzoyl, 3-Sulfonamido-4-chlor-5-[(furyl)-amino]-benzoyl, 2,3-Dichlor-4-(ß-phenyl-acryloyl)-phenoxy-acetyl, Pivaloyl, $C_1$-$C_2$-Alkylaminocarbonyl, $C_1$-$C_2$-Alkylaminothiocarbonyl, Pivaloylmethoxy oder den Rest

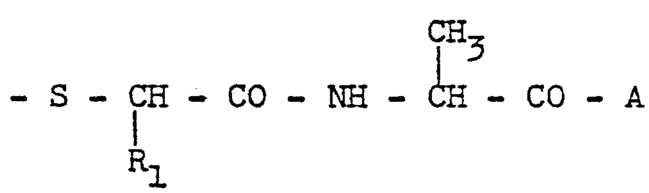

;

A den Rest einer der α-Aminosäuren

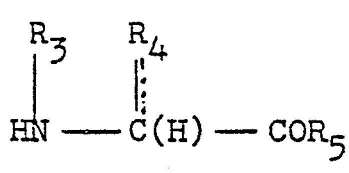

oder

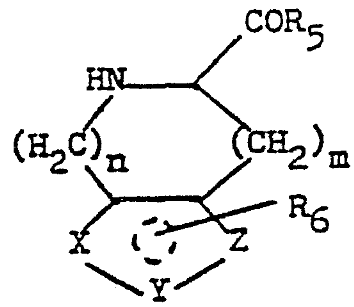

$R_3$ Wasserstoff, $C_1$-$C_4$-Alkyl, gerade oder verzweigt, $C_5$-$C_7$-Cycloalkyl, wobei ein Phenylring ankondensiert sein kann, Phenyl, Phenyl-$C_1$-$C_4$-alkyl, einen Hetero-$C_1$-$C_4$-alkylrest, wobei der Heterocyclus aus einem 5- oder 6-Ring mit 1 - 2 der Heteroatome O, S oder N besteht,

$R_4$ Wasserstoff, $C_1$-$C_4$-Alkyl, gerade oder verzweigt, Phenyl, Phenyl-$C_1$-$C_4$-alkyl oder einen Hetero-$C_1$-$C_4$-alkylrest, wobei der Heterocyclus aus einem 5- oder 6-Ring mit 1 - 2 der Heteroatome O, S oder N besteht,

$R_3$ und $R_4$ zusammen bilden mit dem N- und dem C-Atom einen 5-, 6- oder 7-gliedrigen Ring, der gesättigt sein oder eine Doppelbindung enthalten kann und der gegebenenfalls durch Oxo, Dimercaptoethylene oder ein oder zwei Hydroxy oder Methoxygruppe substituiert ist, oder einen 4-, 5- oder 6-gliedrigen Ring, der ein oder zwei weitere der Heteroatome O, S oder N enthält;

$R_5$ OH, $C_1$-$C_4$-$\omega$-Hydroxyalkoxy, $C_1$-$C_4$-Alkoxy, Phenyl-$C_1$-$C_4$-alkoxy, $C_1$-$C_4$-Alkylamino, Di-($C_1$-$C_4$-alkyl)-amino, eine der Gruppen

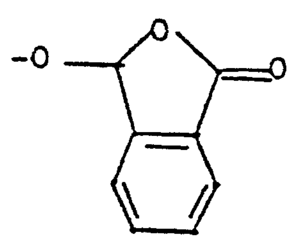

,

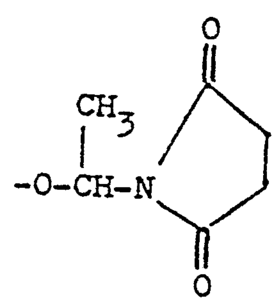

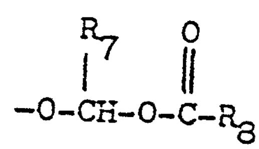

oder

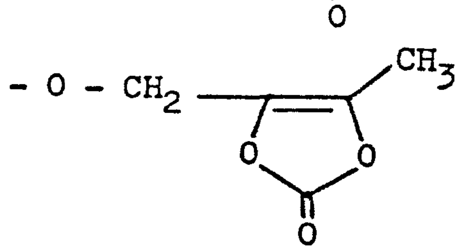

oder eine α-Aminosäure, die peptidartig mit der CO-Gruppe des Moleküls verknüpft ist;

$R_6$ $C_1$-$C_4$-Alkyl, $C_1$-$C_3$-Alkenyl, $C_1$-$C_3$-Alkinyl, gerade oder verzweigt, Hydroxy, Nitro, Amino, $C_1$-$C_4$-Alkoxy, Mercapto, $C_1$-$C_4$-Alkylthio, Hydroxy-$C_1$-$C_4$-alkyl, Mercapto-$C_1$-$C_4$-alkyl, F, Cl, Br, Amino-$C_1$-$C_4$-alkyl, Sulfonamido, Methylendioxy, Fluor-$C_1$-$C_4$-alkyl, Chlor-$C_1$-$C_4$-alkyl, Brom-$C_1$-$C_4$-alkyl, Cyano oder Trifluormethyl;

$R_7$ Wasserstoff oder Methyl;

$R_8$ $C_1$-$C_4$-Alkyl, gerade oder verzweigt, wobei der Alkylrest durch F, Cl, Br, $CF_3$, Phenyl oder Pyridyl substituiert sein kann;

X, Y und Z O, S, $CR_{10}$, $CHR_{10}$,

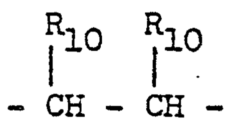 oder 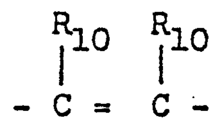 ,

mit der Maßgabe, daß nur einer der Reste X, Y und Z O, S

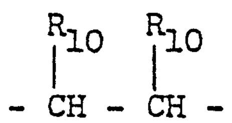 oder 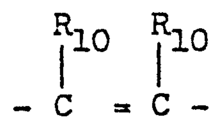 und

einer oder zwei der Reste X, Y und Z $NR_9$ bedeuten können;

$R_9$ Wasserstoff oder $C_1$-$C_4$-Alkyl, gerade oder verzweigt;

$R_{10}$ Wasserstoff, oder zusammen mit einem vicinal stehenden Rest $R_{10}$ einen Phenylring oder, für m und n = 1, dessen Dihydroform mit der Doppelbindung in Konjugation zur C-terminalen Carboxygruppe und

m und n jeweils 0,1 oder 2, wobei die Summe aus m und n 1 oder 2 ist, bedeuten und deren Salze.

2. Aminosäurederivate nach Anspruch 1, dadurch gekennzeichnet, daß
   $R_1$ H, Phenyl, Benzyl oder Phenethyl,
   $R_2$ H, Acetyl oder 3-Sulfonamido-4-chlor-benzoyl und
   A Prolin bedeutet
   und deren Salze.

3. N-[N-(3-Phenyl-2-S-mercaptopropanoyl)-S-alanyl]-S-prolin und dessen Salze.

4. Aminosäurederivate nach den Ansprüchen 1 bis 3, dadurch gekennzeichnet, daß die asymmetrischen Kohlenstoffatome in S-Konfiguration vorliegen.

5. Verfahren zur Herstellung von Aminosäurederivaten der allgemeinen Formel I nach Anspruch 1, dadurch gekennzeichnet, daß man
   a) eine Säure der allgemeinen Formel

$$R_1 - \underset{}{\overset{SR_2}{\overset{|}{CH}}} - COOH \qquad (II)$$

worin
$R_1$ und $R_2$ die oben angegebene Bedeutung besitzen, mit einem Dipeptid der allgemeinen Formel

$$H_2N - \overset{CH_3}{\overset{|}{CH}} - CO - A \qquad (III)$$

worin
A die oben angegebene Bedeutung besitzt, kondensiert, oder daß man

b) eine Verbindung der allgemeinen Formel

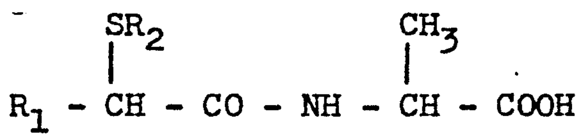

(IIa)

worin
$R_1$ und $R_2$ die oben angegebene Bedeutung haben, mit einer Aminosäure A kondensiert, wobei A die oben angegebene Bedeutung besitzt, oder daß man

c) eine Verbindung der allgemeinen Formel

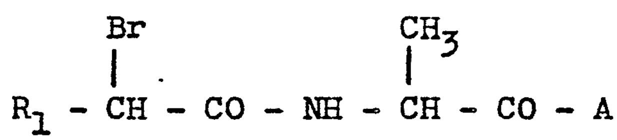

(V)

worin
$R_1$ und A die oben angegebene Bedeutung besitzen, mit einer Mercaptoverbindung der allgemeinen Formel

$$HS - R_2 \qquad (VI)$$

worin
$R_2$ die oben angeführte Bedeutung hat, umsetzt, oder daß man

d) eine Verbindung der allgemeinen Formel

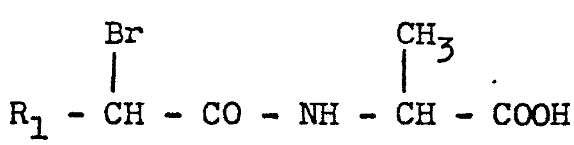

worin
$R_1$ die oben angeführte Bedeutung besitzt, mit einer Aminosäure A kondensiert, wobei A die vorstehend angegebene Bedeutung besitzt, und das so erhaltene Kondensationsprodukt der allgemeinen Formel V mit einer Mercaptoverbindung der allgemeinen Formel VI umsetzt, oder daß man

e) eine Verbindung gemäß Formel I, in welcher $R_2$ $C_1$-$C_4$-Acyl oder Benzoyl ist, zur entsprechenden Verbindung gemäß Formel I, in welcher $R_2$ Wasserstoff ist, verseift, oder daß man

f) eine Verbindung gemäß Formel I, in welcher $R_2$ Wasserstoff ist, mit einer Verbindung der Formel B-$R_2$, in der $R_2$ die obigen Bedeutungen (außer Wasserstoff) hat und B eine leicht abspaltbare Gruppe ist, zur entsprechenden Verbindung gemäß Formel I, in welcher $R_2$ kein Wasserstoff ist, umsetzt

und daß etwa vorhandene Schutzgruppen auf einer geeigneten Stufe der Verfahren a), b), c), e) oder f) abgespalten werden,
und daß man gegebenenfalls ein so erhaltenes Endprodukt der allgemeinen Formel I in ein Salz überführt, oder ein so erhaltenes Endprodukt der allgemeinen Formel I, in der $R_5$ -OH ist, in einen Ester gemäß Formel I überführt.

6. Verfahren zur Herstellung dimerer Verbindungen der allgemeinen Formel I, worin $R_2$ die Gruppe

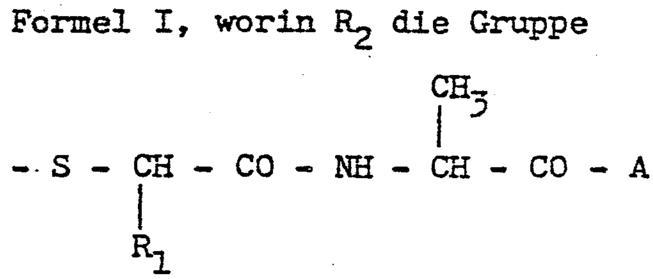

bedeutet und die übrigen Reste die in Anspruch 1 angegebene Bedeutung besitzen, dadurch gekennzeichnet, daß man eine Verbindung der allgemeinen Formel I, in der $R_2$ Wasserstoff bedeutet, mit einem Oxydationsmittel behandelt, und daß man gegebenenfalls eine so erhaltene dimere Verbindung in ein Salz überführt.

7. Pharmazeutische Zubereitungen, enthaltend als Wirkstoff eine oder mehrere Verbindungen der Formel I oder deren Salze in Kombination mit üblichen Hilfs- und/oder Trägerstoffen.

8. Pharmazeutische Zubereitungen, enthaltend als Wirkstoff eine oder mehrere Verbindungen der allgemeinen Formel I in Gemisch mit anderen bekannten Saluretica beziehungsweise Diuretica und/oder Antihypertonica.

9. Verfahren zur Herstellung von pharmazeutischen Präparaten gemäß Anspruch 7, dadurch gekennzeichnet, daß man Verbindungen der allgemeinen Formel I mit üblichen galenischen Hilfs- und/oder Trägerstoffen zu üblichen pharmazeutischen Anwendungsformen verarbeitet.

10. Verwendung der Verbindungen nach Anspruch 1 als Hypotensiva.